# EUROPEAN PATENT SPECIFICATION

(11) **EP 3 374 897 B1**
(45) Date of publication and mention of the grant of the patent: **12.03.2025**
(21) Application number: 16802214.3
(22) Date of filing: 11.11.2016
(51) Int. Cl.: G16H 10/60, G16H 15/00, G16H 20/13, G16H 20/17

(54) **MEDICATION ADMINISTRATION AUDITING SYSTEMS AND METHODS**
MEDIKAMENTENVERABREICHUNGS-AUDITSYSTEME UND -VERFAHREN
SYSTÈMES ET PROCÉDÉS DE VÉRIFICATION D'ADMINISTRATION DE MÉDICAMENTS

(30) Priority: 13.11.2015 US 201562255177 P
(43) Date of publication of application: 19.09.2018
(73) Proprietor: Mayo Foundation for Medical Education and Research, Rochester, MN 55905 (US)
(72) Inventor: D'AMATO, Charles L., Scottsdale, Arizona 85255-8565 (US)
(74) Representative: dompatent von Kreisler Selting Werner - Partnerschaft von Patent- und Rechtsanwälten mbB
(86) International application number: PCT/US2016/061666
(87) International publication number: WO 2017/083748

(56) References cited:
- US-A1- 2011 161 108
- US-A1- 2013 282 392
- US-A1- 2014 149 131
- US-B1- 8 606 596

## Description

### TECHNICAL FIELD

The disclosure relates to auditing of medication administration.

### BACKGROUND

Auditing the use of pharmaceuticals, such as medications, chemicals, and other legally prescribed controlled substances in hospitals and other medical care facilities has become increasingly important. Theft of drugs and controlled substances by healthcare professionals in medical care facilities is a major contributor to the drug diversion problem facing the medical industry. In addition to increasing costs associated with operation of a medical facility, drug diversion can jeopardize patient safety, and pose substantial risk to the individual who is diverting the drugs, as well as co-workers and employers.

US-A-2011/1611108 relates to a system and method for detecting diversion in drug dispensing. Data is received from a number of drug dispensing stations regarding drug dispensing transactions conducted by users of the stations. Based on this data, a diversion score is determined for each user that indicates a relative severity of diversion activity with respect to other users, thereby allowing auditors to conduct further investigations of individual users with certain diversion scores.

US-A-2014/0149131 describes a system and a method for drug diversion tracking including outpatient prescription monitoring. US-B-8 606 596 relates to a medication waste and data collection system that can include one or more of a data collection system with at least one computing system, a medication injection site, and a waste collection system with an inlet port. The data collection system can receive data from the injection site and/or waste collection system and can determine whether medication within a medication container has been diverted. Atypical waste amounts for a caregiver compared to his or her peers may be reported.

### SUMMARY

By way of the present invention there is provided a computer-implemented method for auditing of medication according to claim 1 and a medication administration auditing system of claim 7. Individual embodiments of the invention are the subject matter of the dependent claims.

**In** general, the disclosure is directed to systems and methods for auditing of medication administration. A medication administration auditing system compares medication administration data from, for example, electronic health records (eHR) to medication dispensed from an automated medication dispensing system (AMDS). A non-matching event is categorized as a variance. Results of the analysis may be used for drug diversion prevention, financial charge capture, and/or regulatory compliance, and may also help to improve patient safety.

The method may further include analyzing the transaction data over a period of time to identify trends in the transaction data. The method may further include analyzing the transaction data associated with a specific health care provider.

The at least one processor may be further configured to: for each of a plurality of user identifiers, compare a number of identified variances with a number of identified variances associated with a peer group baseline corresponding to the user.

The at least one processor may be further configured to: for each of a plurality of user identifiers and for each of the plurality of dispense transactions associated with the user identifier, compare the dispensed amount of the medication with an average dispensed amount of the medication associated with a peer group baseline corresponding to the user.

The at least one processor may be further configured to: for each of a plurality of user identifiers and for each of the plurality of waste transactions associated with the user identifier, compare a wasted amount of a medication with an average wasted amount of the medication associated with a peer group baseline corresponding to the user.

The at least one processor may be further configured to: for each of the plurality of waste transactions, receive assay data corresponding to a quantitative assay indicative of an actual wasted amount of the medication; and for each of a plurality of user identifiers and for each of the plurality of waste transactions, compare the actual wasted amount of the medication with an average wasted amount of the medication associated with a peer group baseline corresponding to the user.

The at least one processor may be further configured to: receive infusion medication administration data associated with a plurality of infusion devices, the infusion medication administration data including, for each administration of an infusion medication by the plurality of infusion devices, a user identifier, an infusion medication identifier, a patient identifier, and an actual administered amount of the infusion medication; receive infusion medication waste transaction data associated with a plurality of infusion medication waste transactions performed in an automated medication dispensing system, the infusion medication waste transaction data including, for each of the plurality of infusion medication waste transactions, a user identifier, an infusion medication identifier, a patient identifier, and a wasted amount of an infusion medication; for each administration of an infusion medication by the plurality of infusion devices, compare the administered amount of the medication with a third peer group baseline corresponding to the user identifier; for each of the plurality of waste transactions, compare the wasted amount of the medication with a fourth peer group baseline corresponding to the user identifier; and identify a variance indicative of a potential drug diversion for each one of the comparisons that satisfy a variance threshold.

The details of one or more examples of the present disclosure are set forth in the accompanying drawings and the description below. Other features, objects, and advantages will be apparent from the description and drawings, and from the claims, wherein the scope of protection is defined in the claims.

### BRIEF DESCRIPTION OF DRAWINGS

FIG. 1 is a block diagram illustrating an example medication administration auditing system of the present disclosure.
FIG. 2 is a flow diagram illustrating an example process by which a medication administration auditing system may analyze medication transactions and identify variances which may be indicative of possible drug diversion events.
FIG. 3 is a flow diagram illustrating a more detailed example process by which a medication administration auditing system may analyze medication transactions and identify variances which may be indicative of possible drug diversion events.
FIGS. 4-19B show example reports that may be generated by the medication administration auditing system of the present disclosure.
FIG. 20 is a diagram illustrating an example drug diversion prevention model.
FIG. 21 is another diagram illustrating an example drug diversion prevention model.
FIG. 22 is a flowchart illustrating an example waste assay tracking process.
FIG. 23 is a flow chart illustrating an example drug diversion prevention and identification auditing process.
FIG. 24 is a flow chart illustrating another example drug diversion prevention and identification auditing process.
FIG. 25 is a diagram illustrating an example overall process that may be executed by a medication administration auditing system.

### DETAILED DESCRIPTION

In general, a medication administration auditing system (also referred to herein as "auditing system" or "medication auditing system" or "MAAP") is an auditing tool developed to provide users with fast, concise medication administration information. For example, the auditing system may analyze data from electronic health records (eHR) and/or data from an automated medication dispensing system (AMDS). The concepts may be employed with any eHR and/or AMDS platforms. The auditing system may be used by a healthcare institution as an effective auditing tool for drug diversion prevention. In terms of financial auditing, the auditing system may be utilized to verify medication documentation and consequently medication charge capture in institutions utilizing the charge on administration billing scenario, or can be used to verify billing/credit on charge on dispense systems. The auditing system may confirm proper documentation of all medications scheduled for administration and may verify medication administration compliance. The auditing system may perform fast, accurate audits in real-time, focus auditors on outliers of specific clinical and administrative data in each application, provide long term tracking, generate reports specific to drug diversion and decrease risk associated with drug diversion. The auditing system may be utilized for controlled and/or non-controlled medication. The medication auditing system may include algorithms specific to different applications within a healthcare institution, resulting in a more precise identification of possible drug diversion events. The auditing system uses multiple variables of activity and processes and does not require teams of individuals to interpret multiple reports for possible diversion events. The auditing system is powerful in the sense that it may review and analyze large volumes of medication administration activities precisely with minimal human interface.

The auditing system may further be utilized in conjunction with a waste retrieval and medication assay process, which may thus provide further barriers to drug diversion. The auditing system may define expected waste, waste volumes and concentrations of medication that may be verified with assay. The auditing system uses multiple sources and analyzes data over time to "triangulate" potential diversion.

The medication administration auditing system may be used in any area within a healthcare institution having drug diversion potential, including, for example, anesthesia medication administration, nursing medication administration, outpatient prescription medication administration, medication administration in procedural settings (such as Endoscopy, Emergency Room, Catherization Lab, Operating Room Surgical and Interventional Radiology), Patient Controlled Analgesia (PCA) and Epidural administration, auditing of the dispensing of medication in pharmacy settings and also auditing of medications used for compounding within pharmacy settings, etc.

The medication administration auditing systems and methods may be designed to meet clinical practice needs, provide the ability to electronically account for all medication, identify variances and/or discrepancies, and to perform longitudinal tracking over time. The medication administration auditing systems and methods may further provide for a chain of custody throughout the medication retrieval and waste assay process. The medication administration auditing may, by identifying and/or preventing drug diversion, increase the security of medications in healthcare settings and increase patient safety.

In some examples, the medication administration auditing systems and/or methods may allow for independent user disposition of a controlled substance. In other words, rather than requiring a "witness" to waste a controlled substance, the processes and procedures underlying the medication administration auditing system provide confirmation that a medication or other controlled substance was properly disposed of. This may simplify and increase efficiency of the medication waste process because a user need not find an administrator or other witness to verify their controlled substance waste. It may also increase the likelihood that users will properly deposit controlled substance waste in designated waste bins rather than dispose of controlled substances in sinks, toilets, or trash. It may further identify and/or prevent diversion by two colleagues working together.

Waste retrieval and assay processes defined by the medication administration auditing systems and/or methods require that a drug diversion prevention team retrieve and analyze controlled substance waste via a chain of custody process. A waste assay is performed the controlled substance waste samples. The analysis may include, for example, a comparison of the actual wasted volume of the medication with an expected waste volume, a comparison of the actual identified wasted substance with an expected waste medication, and/or a comparison of the actual wasted medication concentration with an expected wasted medication concentration.

The medication administration auditing systems and/or methods further include longitudinal tracking and analysis of medication administration auditing information over time. This longitudinal analysis may identify patterns indicative of one or more drug diversion events. The system may review data by user, employee type or job role, medication, dispensing location, patient, timeframe, practice area/department, geographic location of the healthcare facility, etc. Long term tracking may also allow feedback for education and practice improvement when auditing reveals issues that are not instances of drug diversion.

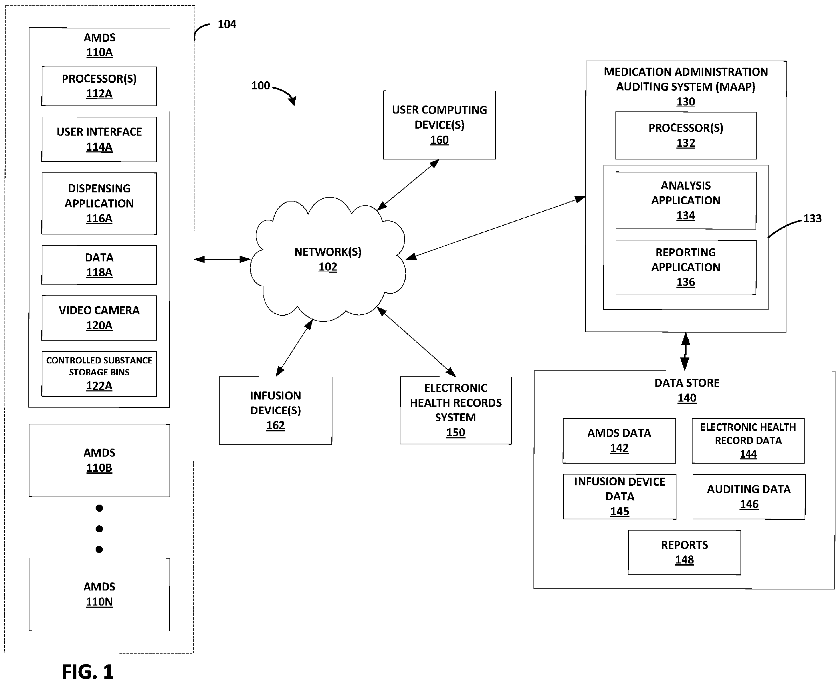

FIG. 1 is block diagram illustrating an example environment 100 in which a medication administration auditing system may be used. System 100 includes an automated medication dispensing system 104, an electronic health record system 150, one or more infusion device(s) 162, and a medication administration auditing computing system 130.

Automated medication dispensing system 104 includes one or more automated medication dispensing stations (AMDS) 110A-110N that may be located throughout the healthcare facility. AMDS 110A-110N include drug storage devices or cabinets that electronically dispense medications in a secure, controlled fashion. AMDS 110A-110N may be stocked by centralized or decentralized pharmacies. Before dispensing any medication, AMDS 110A-110N may require user identifiers, passwords and/or biometric input (fingerprint, facial recognition, etc.) for user authentication. AMDS 110A-110N and system 104 may electronically record and track users accessing the system, patients for whom medications are dispensed, medications dispensed, wasted medications, maintain inventory data, and provide usage data and analytics. System 104 may include different types of dispensing stations designed for different applications (e.g., anesthesia, nursing, pharmacy, OR, etc.).

AMDS 110A-110N communicate via network(s) 102 with medication auditing computing system 130. Computing system 130 may be, for example, one or more remote servers or other computing systems capable of auditing medication administration and identifying possible diversion events as described herein. Network(s) 102 may include, for example, one or more of a dial-up connection, a local area network (LAN), a wide area network (WAN), the Internet, a cell phone network, satellite communication, or other means of electronic communication. The communication may be wired or wireless. System 130 may receive data or otherwise communicate with AMDS 110A-110N on a periodic basis, in real-time, upon request of any of user devices 110A-110N, upon request of system 130, or at any other appropriate time. System 130 may further communicate with an electronic health record system 150 to obtain electronic health record data for analysis during the medication auditing process.

Infusion device(s) 162 are electronically controlled medical devices that deliver fluids, such as nutrients and medications, into a patient's body in controlled amounts. Infusion devices 162 may include for example, any type of infusion pump, such as large volume pumps, patient-controlled analgesia (PCA) pumps, elastomeric pumps, syringe pumps, enteral pumps, and insulin pumps. The infusion device(s) 162 include electronic storage media that records the infusion device history, including time and date, user, patient, medication, volume, dosage, pressure, and any other device related data.

Computing system 130 includes one or more processors 132 and storage media 133. Storage media 133 includes an analysis application 134 and a reporting application 136. Analysis application is a software module that, when executed by processor(s) 132, compares AMDS data concerning each medication dispensing event with electronic health record data. Analysis application 134 may further identify possible diversion events based on the analysis. Reporting application 136 is a software module that, when executed by processor(s) 132, may generate reports at the request of a user concerning the medication auditing analysis, or may automatically generate reports or alerts on a periodic basis. Reporting application 136 may further generate reports or alerts as needed based on the results of the analysis.

Each AMDS 110A-110N may include one or more processors 112A-112N, a user interface 114A-114N, a dispensing control module 516A-516N, and data storage 118A-118N, respectively. Each user interface 114A-114N may include components that are input-only (such as a physical keyboard, microphone, touch sensor, touchpad, camera, mouse, stylus, barcode scanner, fingerprint sensor, biometric sensors, etc.), components that are output-only (electronic display, audio speaker, printer, etc.), and some components that include both input and output functionality (touchscreen, etc.). Each AMDS 110A-110N may further include a video camera 120A-120N, and one or more controlled substance storage bins 122A-122N, respectively.

Each example medication dispensing control module 116A-116N is a software module that, when executed by processor(s) 112A-112N, respectively, permits a user, via user interface 114A-114N, to dispense medication(s) from the secure AMDS cabinet. For example, control module 116A-116N may oversee secure authentication of a user and manage controlled access to the appropriate drawers, pockets, or bins (indicated generally by reference numerals 122A-122N in FIG. 1) where the requested medications are stored and/or wasted within the cabinet. Control module 116A-116N may further store and analyze AMDS data 118A.

Auditing computing system 130 receives AMDS data 118 from the AMDS 110A-110N and/or system 104 via networks 102. The AMDS data 118 received from AMDS 110A-110N may be stored at a data store or other data storage media 140, for example as AMDS data 142. Data store 140 may further include electronic health record data 144, infusion device data 145, auditing data 146, and reports 148. Auditing data 146 may include any data generated by medication auditing system 130 during analysis of the AMDS data 142, infusion device data 145 and electronic health record data 144 received from electronic health record system 150. Auditing data 146 may include, for example, data concerning any possible diversion events. The auditing data 146 may also provide a quality improvement component by identifying practice and process errors in the medication waste activities of a healthcare facility.

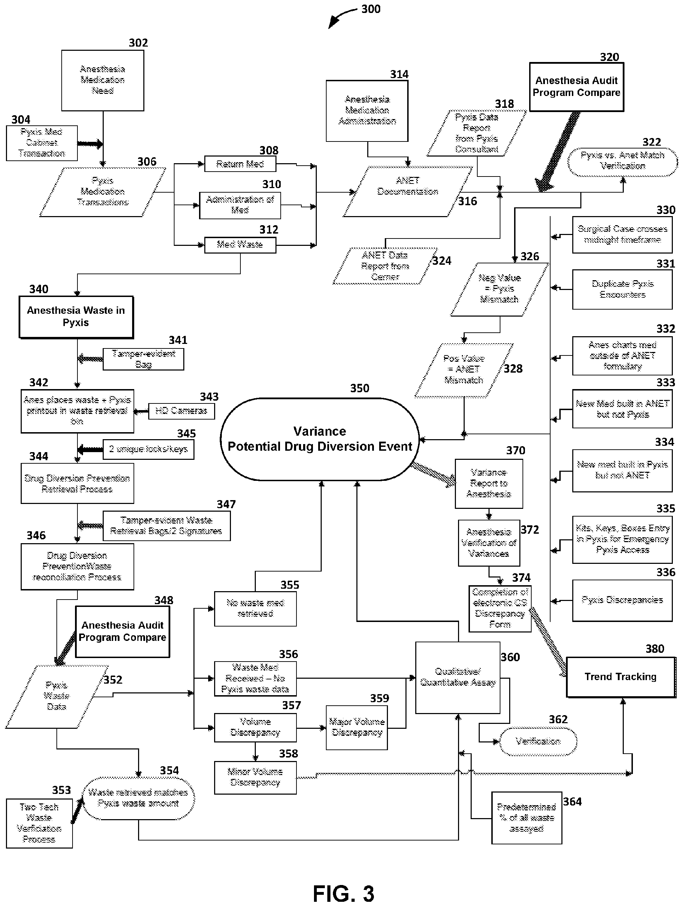

Reporting application 136 may generate a variety of reports to provide users with both qualitative and quantitative data regarding auditing of medication administration. The reports may be provided based on a user, a medication-type, a patient, an application or area (such anesthesia, pharmacy, medical-surgical, etc.), an individual or defined group of AMDS, based on day and/or time, etc. The reports may further provide financial information, drug diversion or variance information, track waste retrieval and disposal information, etc. The reports may be transmitted to the appropriate user device(s) 160 for presentation on the associated user interface. The reports may further be manipulated by the user to generate additional reports, as described herein, and may be printed or otherwise output as desired by a user. Example reports that may be generated by medication administration auditing system 130 are shown in FIGS. 3-19.

Reports 148 generated by reporting application 136 may be stored in data store 140. The reports 148 may be accessed by user devices 160 at the time they are generated or at a later time over network(s) 102. One or more of the reports 148 may be downloaded and stored on a user device 160 or other authorized computing device, printed out in hard copy or further communicated to others as desired.

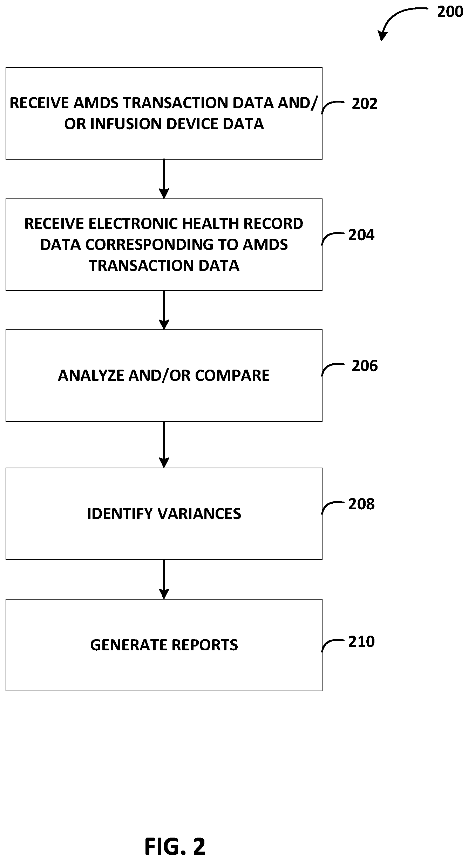

FIG. 2 is a flow diagram illustrating an example process (200) by which a medication administration auditing device/system, such as medication auditing computing system 130 of FIG. 1, may analyze medication transactions and identify variances which may be indicative of possible drug diversion events.

The computing device receives AMDS transaction data associated with medication transactions (202). The AMDS transaction data may include any information related to dispensation or wasting of a medication, including but not limited to the name of the medication, transaction type (e.g., removed, wasted, returned to stock) the amount of the medication, user name or identifier, witness name or identifier, patient name or identifier, time and date, prescribing physician name or identifier, AMDS identifier and/or location, or any other information relevant to a medication dispense or waste transaction.

The computing device further receives electronic health record (eHR) data corresponding to the AMDS transaction data (204). The computing device may query an eHR system or database, such as eHR system 150 of FIG. 1, for the eHR data. The computing device analyzes the data by, for example, comparing the AMDS data to the corresponding eHR data (206). A non-matching event is categorized as a variance (208). In some examples, the system may generate one or more reports including the results of the analysis. Results of the analysis may be used for drug diversion prevention, financial charge capture, and/or regulatory compliance, and may also help to improve patient safety.

The computing device receives AMDS transaction data associated with medication dispensing and/or waste transactions (202). The AMDS transaction data may include any information related to dispensation or wasting of a medication, including but not limited to the name of the medication requested or dispensed or wasted, transaction type (e.g., removed, wasted, returned to stock) the amount of the medication, user name or identifier, witness name or identifier, patient name or identifier, time and date, prescribing physician name or identifier, AMDS identifier and/or location, waste volume, amount, or concentration, AMDS waste identifier and/or location, time and date, or any other information relevant to a dispense transaction.

The computing device further receives electronic health record (eHR) data corresponding to the AMDS transaction data (204). The computing device may query an eHR system or database, such as eHR system 150 of FIG. 1, for the eHR data. The computing device analyzes the data by, for example, comparing AMDS data concerning medication dispensed from the (AMDS) to the corresponding eHR data (206). A non-matching event is categorized as a variance (208). The computing device may further analyze the data (206) by analyzing the AMDS data concerning the medication dispensed and/or wasted from the AMDS over time based on user, mediation, type of user, location, timeframe, practice, etc. In some examples, the system may generate one or more reports including the results of the analysis. Results of the analysis may be used for drug diversion prevention, financial charge capture, and/or regulatory compliance, and may also help to improve patient safety.

FIG. 3 is a flow diagram illustrating a more detailed example process (300) by which a medication administration auditing system, such as system 130 of FIG. 1, may analyze medication transactions and identify variances which may be indicative of possible drug diversion events. Example process (300) is described as being specific to auditing of anesthesia medication administration; however, it shall be understood that the same or similar processes may be applied to administration of other types of medication in other areas of a healthcare institution, and that the disclosure is not limited in this respect.

When an anesthesia medication is needed (302), a user executes an AMDS transaction (304, 306). In this example, the AMDS is a Pyxis^{™} automated medication dispensing system; however, it shall be understood that any automated mediation dispensing system may be used, and that the disclosure is not limited in this respect. The transaction may include return of a medication to stock transaction (308), administration of a medication transaction (310), or a medication waste transaction (312). The AMDS data (314, 318) and the electronic health records corresponding to the AMDS data are obtained (316, 324). The system compares the AMDS data and the corresponding eHR data (320, 322). If the data does not match (326, 328), the mismatch is categorized as a variance indicating a possible drug diversion event (350). Example situations which may result in a mismatch are illustrated in FIG. 3 (330-336).

In the event of a medication waste transaction (340), the process for wasting a medication is followed (341-346). The AMDS data regarding the wasted medication (352) and the expected amount of waste are compared (348). Any discrepancy may be identified as a mismatch (355, 356, 357, 358, 359). The method may include a qualitative or quantitative assay (360) and the results of the assay may be verified (362). In a two-tech waste retrieval verification process (353) if the waste retrieved matches the AMDS waste amount (354) an assay may be performed for verification purposes (360, 362). Any mismatches may be identified as a variance indicating a possible drug diversion event (350).

The system may generate reports (370) which may be sent to the appropriate area or persons for verification (372). Any discrepancies/variance may require completion of an electronic discrepancy form (374). The system may further track trends over time and generate one or more reports concerning those trends (380).

Examples of specific processes by which a medication administration auditing system may analyze AMDS data (e.g., as indicated by reference numeral 206 in FIG. 2) will now be described. For example, a process for a nursing auditing program, an anesthesia monitoring program, a procedural auditing program, a Controlled Analgesia (PCA) and Epidural administration auditing program, and a pharmacy auditing program will now be described. Although specific examples are described herein, it shall be understood that the same or similar processes may also be applied to any area in which medication administration is performed, and that the disclosure is not limited in this respect.

An example nursing auditing program may utilize the Health Level Seven (HL7) data stream from the electronic healthcare administration record (eHR). The program compares and analyzes clinical documentation to medication administration obtained from automated medication dispensing systems (AMDS).

The program compares nursing medication administration documentation (charting) to the net of medication dispensed from an AMDS removing comparisons that match, resulting in variances to expected practice for each category. The cross-view sum of each category may direct an auditor to review for drug diversion. The example nursing auditing program may further include one or more of the following functions during the analysis:
- Determine the net amount of medication dispensed from the AMDS for comparison, accounting for remove, return to stock, return to external bin (e.g., return bins that provide secure storage for previously removed, unopened medications that will be returned for inspection and return to inventory), cancel, and waste transactions.
- Determine any medication removed from an AMDS and not charted as administered.
- Determine any medication removed from an AMDS where the net amount removed does not equal the amount charted as administered.
- Determine the net amount of medication documented in eHR.
- Determine when medications have been administered outside specified time periods from removal from AMDS.
- Determine when medications have been removed from an AMDS and are fully wasted.
- Determine total amount of net removals and compare to colleagues.
- Determine total amount of waste transactions and compare to colleagues.
- Determine total amount of AMDS cancel transactions and compare to colleagues.
- Determine medication not wasted upon immediate medication removal from AMDS.
- Determine when a nurse that removed a medication is not the primary person wasting the medication.
- Determine when a medication removed from an AMDS is not the nurse charting the medication.
- Total AMDS discrepancies by a specific user.
- Determine if a medication was charted, but does not have a corresponding medication removal from an AMDS.
- May perform analysis of medications administered to check for clinical appropriateness including, but not limited to utilizations of pain scores, blood pressure, heart rate, respirations, frequency of administration and amount of time elapsed between repetitive medication dosing.
- Determine medications that should have been sent to AMDS "Return to External Bin".
- May be used with a waste retrieval process to determine expected medication waste, volume and concentration. May be utilized with an electronic waste assay process to compare expected waste quantitatively.

An example anesthesia auditing program may utilize the HL7 data stream from the electronic healthcare administration record (eHR). The program compares and analyzes Anesthesia clinical documentation to Anesthesia medication administration obtained from electronic medication dispensing systems (AMDS).

The program compares Anesthesia medication administration documentation (charting) to the net of medication dispensed from an AMDS removing comparisons that match. The data from a number of different categories is then cross-compared using algorithmic analyses pinpointing variances and areas of potential diversion. The example anesthesia auditing program may further include one or more of the following functions:
- Determine the net amount of medication dispensed from the AMDS for comparison, accounting for remove, return to stock, return to external bin, cancel, and waste transactions.
- Determine any medication removed from an AMDS and not charted as administered.
- Determine any medication removed from an AMDS where the full, net amount removed does not equal the amount charted as administered.
- Determine the net amount of medication documented in eHR.
- Determine when medications have been administered outside specified time periods from removal from AMDS.
- Determine when medications have been removed from an AMDS and are fully wasted.
- Determine total amount of net removals and compare to colleagues.
- Determine total amount of waste transactions and may be compared to colleagues.
- Determine total amount of AMDS cancel transactions and compare to colleagues.
- Determine when a medication removed from an AMDS is not the same person charting the medication.
- May total AMDS discrepancies by a specific user.
- May determine if a medication was charted, but does not have a corresponding medication removal from an AMDS.
- Perform analysis of medications administered to check for clinical appropriateness including, but not limited to utilizations of pain scores, blood pressure, heart rate, respirations, frequency of administration and amount of time elapsed between medication dosing.
- Determine medications that should have been sent to AMDS "Return to External Bin".
- May be used with a waste retrieval process to determine expected medication waste, volume and concentration.
- May be utilized with an electronic waste assay process to compare expected waste quantitatively.
- Provides a variance report for Anesthesia auditing.
- Tracks all variances for long term tracking and pattern identification. The long-term data may be sorted in multiple ways to determine the occasional diverter. The information is commonly utilized with the waste retrieval process information to determine diversion behavior.
- Provides a Waste Retrieval Report. The report indicates every waste that the auditor should have for physical comparison. It includes all information necessary for verification and has separate reports and tracking for unaccounted waste and volume variance tracking.

An example procedural auditing application may be very similar to the anesthesia application. The procedural auditing application may utilize the HL7 data stream from the electronic healthcare administration record (eHR). The program compares and analyzes clinical documentation in procedural areas to medication administration obtained from electronic medication dispensing systems (AMDS) in a procedural setting. Medication dispensed in these areas is obtained from an AMDS that allow a user to remove any medication in the cabinet and the removal of the medication is not physically restricted. Examples of procedural areas would include Endoscopy, Emergency Room, Catherization Lab, OR Surgical and Interventional Radiology.

Program Functionality - Compares medication administration documentation (charting) in a Procedural Practice to the net of medication dispensed from an AMDS removing comparisons that match. The resulting output of variances is cross-compared using algorithmic analyses, pinpointing areas of potential diversion. The example procedural auditing program may further include one or more of the following functions:
- Determine the net amount of medication dispensed from the AMDS for comparison, accounting for remove, return to stock, return to external bin, cancel, and waste transactions.
- Determine any medication removed from an AMDS and not charted as administered.
- Determine any medication removed from an AMDS where the net amount removed does not equal the amount charted as administered.
- Determine the net amount of medication documented in eHR.
- Determine when medications have been administered outside specified time periods from removal from AMDS.
- Determine when medications have been removed from an AMDS and are fully wasted.
- Determine total amount of net removals and compare to colleagues.
- Determine total amount of waste transactions and compare to colleagues.
- Determine total amount of AMDS cancel transactions and compare to colleagues.
- May determine when a nurse that removed a medication is not the primary person wasting the medication.
- May determine when a medication removed from an AMDS is not the same person charting the medication.
- Total AMDS discrepancies by a specific user.
- Determine if a medication was charted, but does not have a corresponding medication removal from an AMDS.
- May perform analysis of medications administered to check for clinical appropriateness including, but not limited to utilizations of pain scores, frequency of administration and amount of time elapsed between repetitive medication dosing.
- Determine medications that should have been sent to AMDS "Return to External Bin".
- May be used with a waste retrieval process to determine expected medication waste, volume and concentration.
- May be utilized with an electronic waste assay process to compare expected waste quantitatively.

An example Patient Controlled Analgesia (PCA) and Epidural administration program may compare the HL7 clinical data of administration to the dispensing of medication including returns and waste and also to an electronic administration history from the pump administration.

Program Functionality - PCA/Epidural administration has very high potential for diversion due to the high volumes and high concentrations of medications utilized. In addition, auditing and accountability for this type of therapy is very difficult. Utilizing an AMDS for dispensing, returns and electronic waste of PCA/Epidural medication, this application may make comparisons of the net amount of drug administered to a patient compared to the sum of expected administration based on the prescriber order. Waste retrieval and assay may be utilized to confirm unused medication. The medication administration pump history may also be compared to the charted administration and the physical administration for medication accountability. The example PCA auditing program may further include one or more of the following functions:
- Compare AMDS removal transactions to prescriber therapy orders verifying appropriate removal of PCA/Epidural medication from an AMDS.
- Determine if a controlled substance was removed from an AMDS without prescriber orders.
- Verify physical waste volumes to expected waste volumes based on prescriber order.
- Verify physical waste volumes to expected waste volumes based on pump administration history.
- Verify pump administration settings to prescriber order settings.
- Verify appropriate removal of pump administration key to prescriber orders for the medication.
- When utilized with a waste retrieval process, expected waste volume may be calculated from the prescriber order and initiation of therapy to AMDS removal and administration pump history.
- When utilized with a medication assay process, medication waste retrieved may be quantitatively verified.

An example Pharmacy controlled substance inventory control cabinet program, involves auditing and inventory control processes of controlled substance medication inventory in the Pharmacy. The hardware device is essentially an AMDS containing all controlled substance inventory in an inpatient pharmacy. Large quantities of controlled substance medication inventory are received and dispensed through this device.

Program Functionality - Application Five audits transactions that involve all AMDS cabinets in a healthcare institution, non-AMDS transactions (patient-specific dispensing), and inventory control. This application verifies transactions that occur in other some of the other MAAS applications. The example pharmacy auditing program may further include one or more of the following functions:
- Verifies correct medication and volume arrival to an AMDS through the use of a compare report.
- Verifies waste and returns to the control cabinet from outdated medication from an AMDS.
- Verifies "Return to Bin" transactions from the other applications of MAAS.
- Provides a tracking mechanism for expired medication.
- Utilizes tracking on broken inventory.
- May be utilized to compare receiving inventory to physical inventory in the cabinet reviewing for variances.
- Through the use of mobile electronic signature devices, receipt of a controlled substance medication may be electronically verified maintaining chain of custody. This application could analyze medication dispensed from the inventory dispense cabinet to the receiver and track and record all information related to the dispensing of that medication.

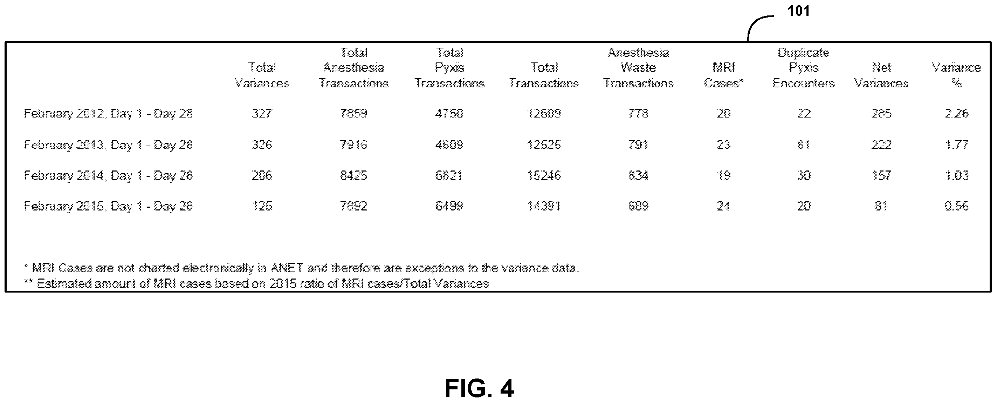

FIG. 4 a table 101 illustrating an example of how use of the medication administration auditing system such as that described herein resulted in a reduction in net variances from 285 (2.26% of all transactions) during the period February 1-28 of 2012, to 222 (1.77% of all transactions) during the period February 1-28 of 2013, to 157 (1.03% of all transactions) during the period February 1-28 of 2014, and to 81 (0.56% of all transactions) during the period February 1-18 of 2015.

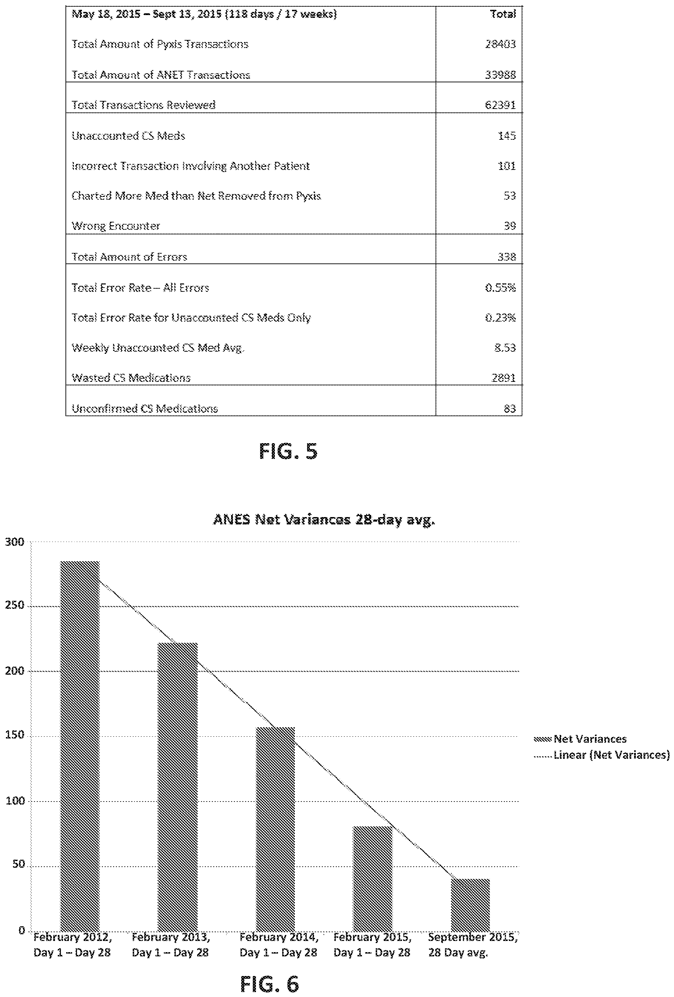

FIG. 5 is an example report for the period May 18, 2015 to September 13, 2015, detailing, for example, the total number of transactions reviewed, the total number of unaccounted for CS (controlled substance) medications, the total amount of errors, and the total number of unconfirmed CS medications detected by the medication administration auditing system described herein.

FIG. 6 is an example graph illustrating a reduction in net variances over time resulting from implementation of the medication administration auditing system described herein.

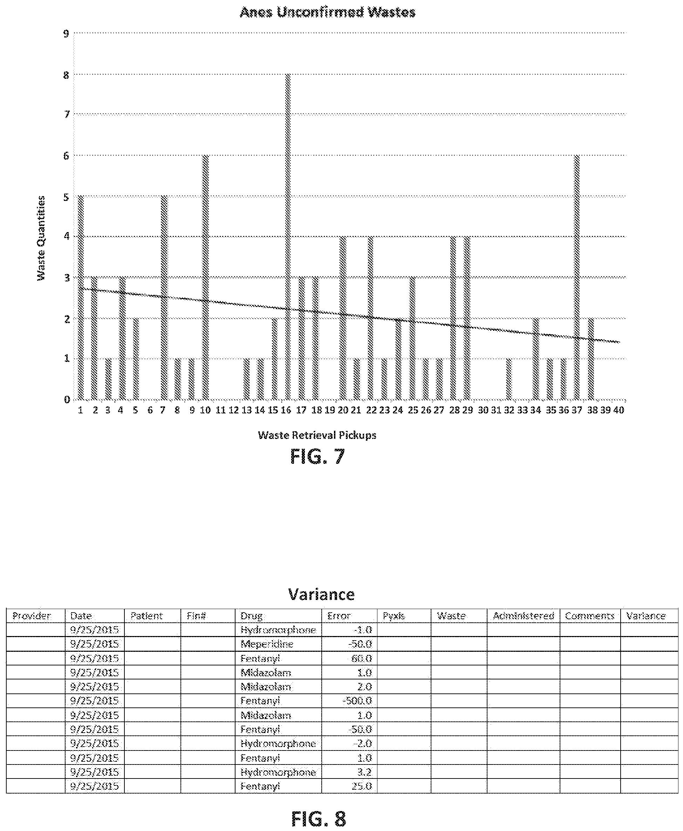

FIG. 7 is an example graph illustrating the quantity of anesthesia unconfirmed CS waste versus waste retrieval pickups.

FIG. 8 is an example table listing variances reported by the medication administration auditing system described herein.

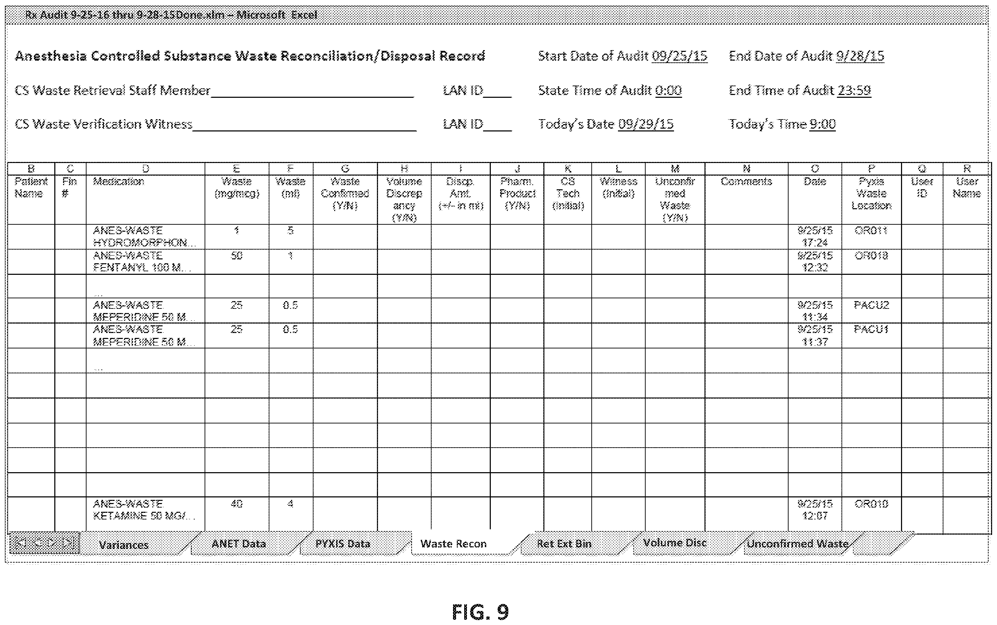

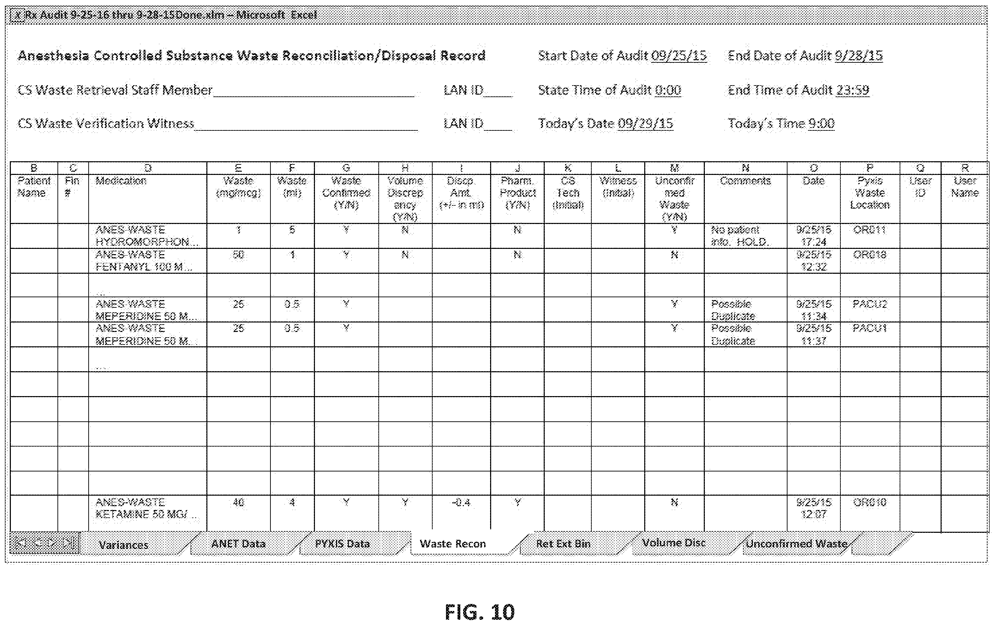

FIG. 9 is an example anesthesia controlled substance waste reconciliation disposal record (blank) and FIG. 10 is the example anesthesia controlled substance waste reconciliation disposal record of FIG. 9 including assay results and comments.

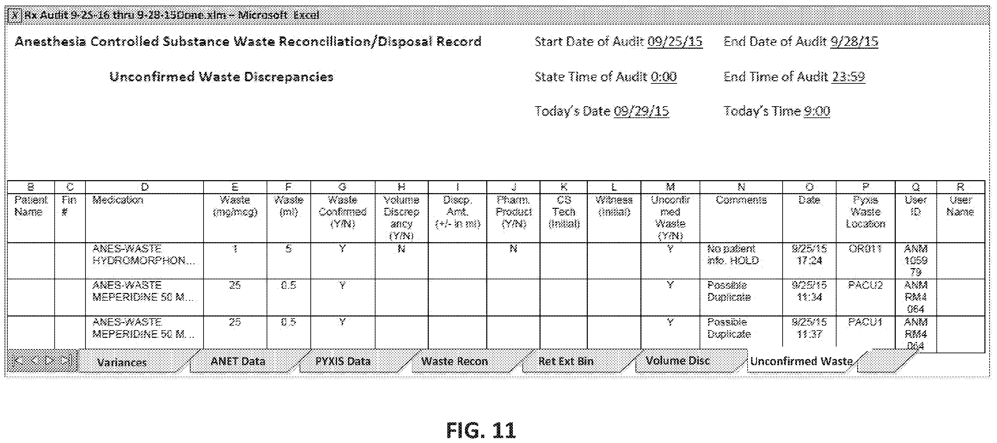

FIG. 11 is an example report of unconfirmed waste discrepancies reported by the medication administration auditing system described herein.

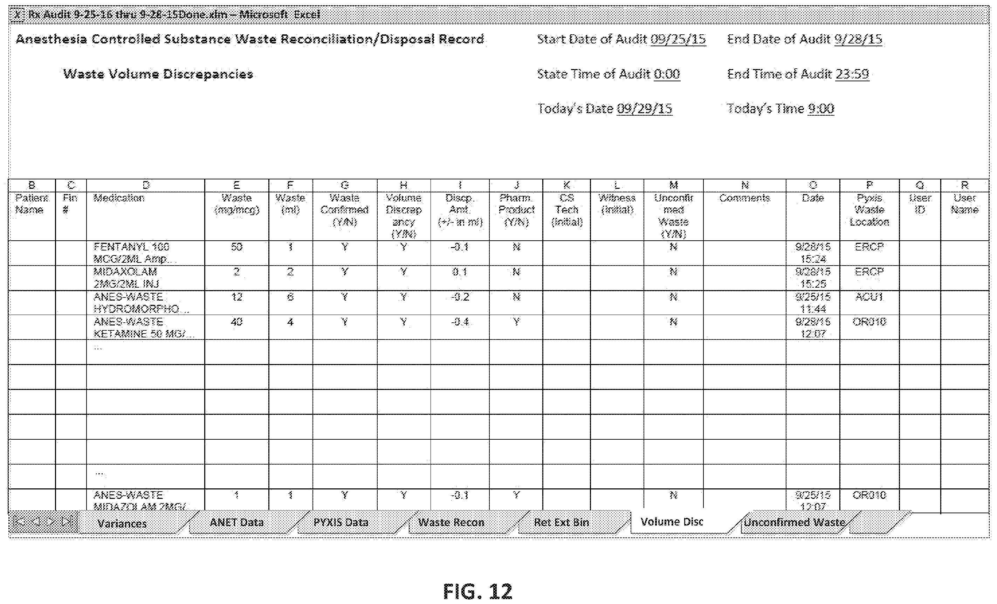

FIG. 12 is an example report of waste volume discrepancies reported by the medication administration auditing system described herein.

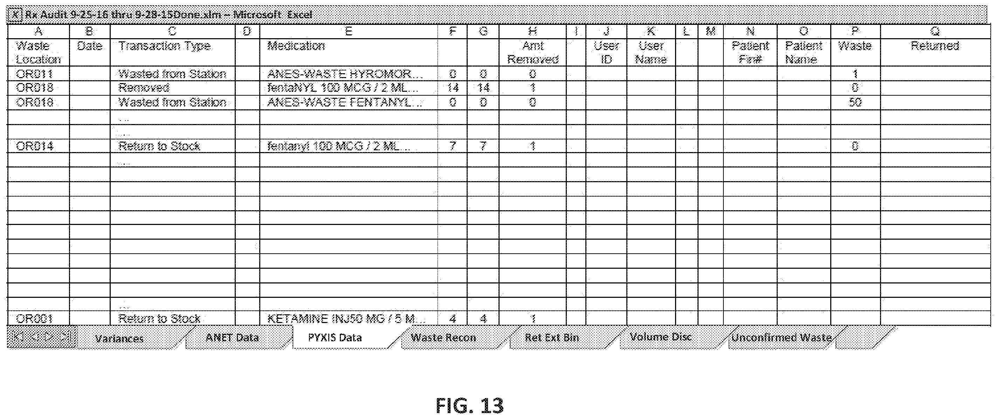

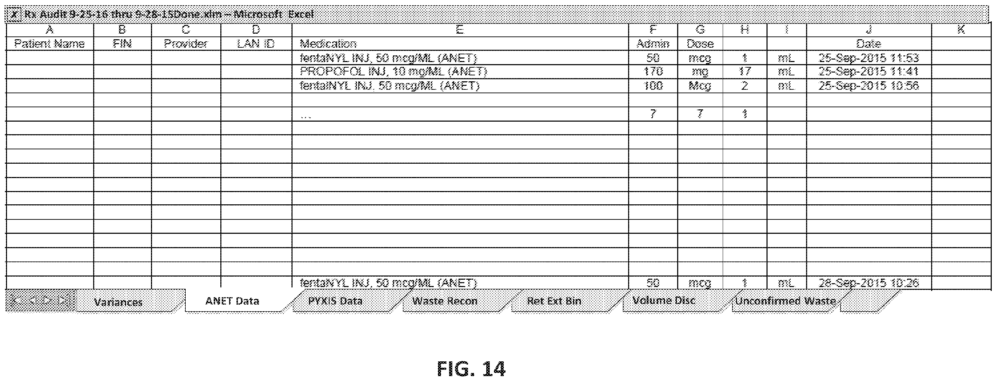

FIGS. 13 and 14 are example reports of AMDS transactions generated by the medication administration auditing system described herein.

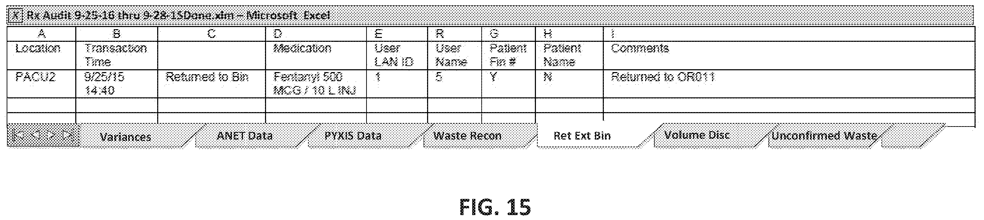

FIG. 15 is an example report of return to external bin transactions (return bins that provide secure storage for previously removed, unopened medications that are returned for inspection and return to inventory) generated by the medication administration auditing system described herein.

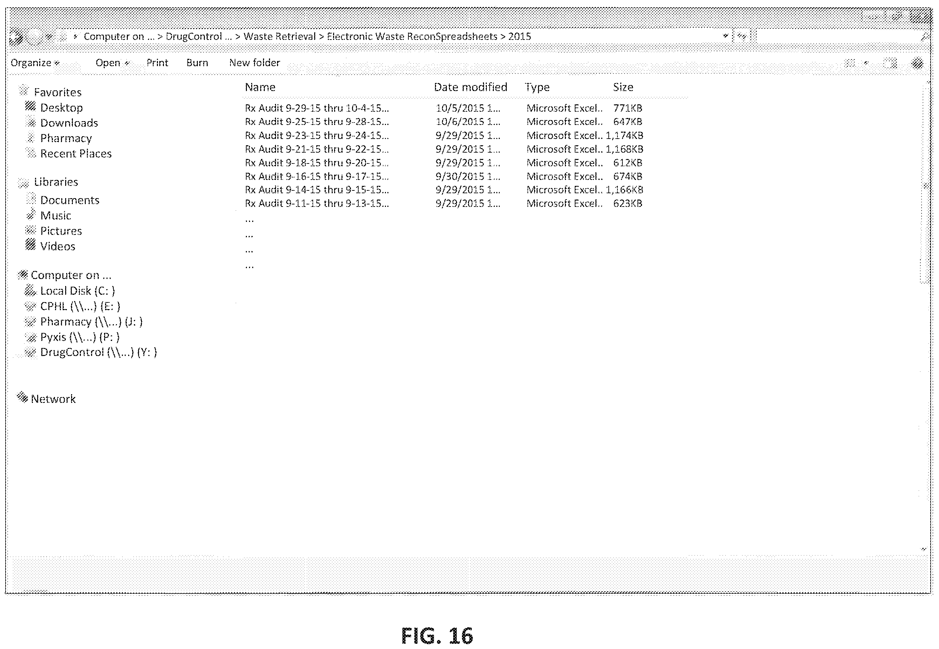

FIG. 16 is an example report showing a list of electronically stored waste reconciliation spreadsheets by date generated by the medication administration auditing system described herein.

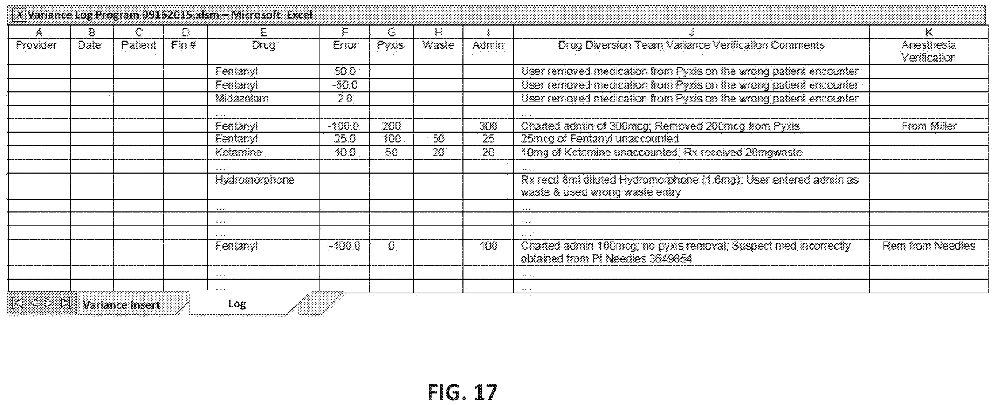

FIG. 17 is an example report of a variance log listing variances and comments from the drug diversion prevention team generated by the medication administration auditing system described herein.

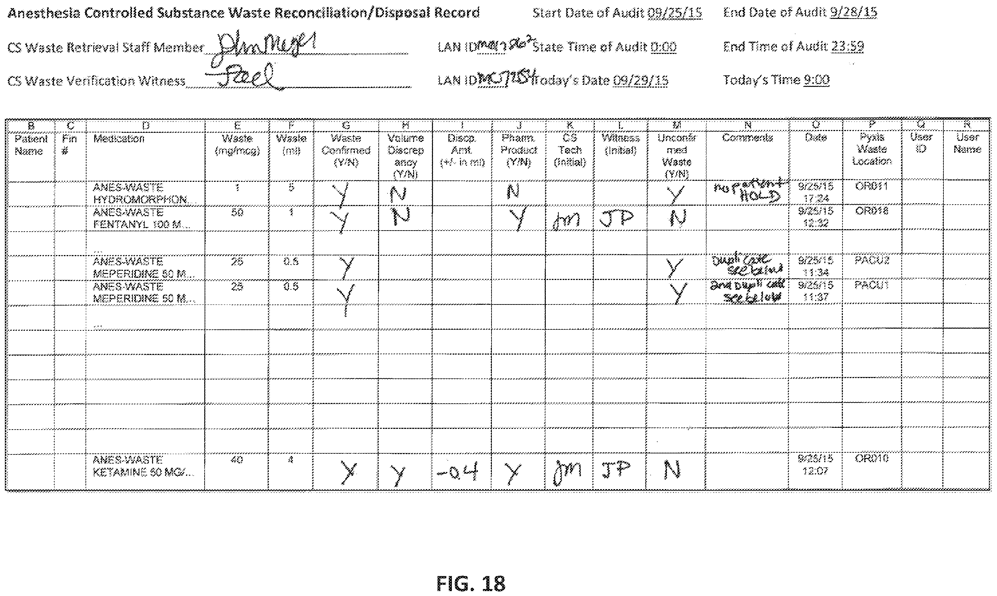

FIG. 18 is an example report of an assay log of the type used with the medication administration auditing system described herein.

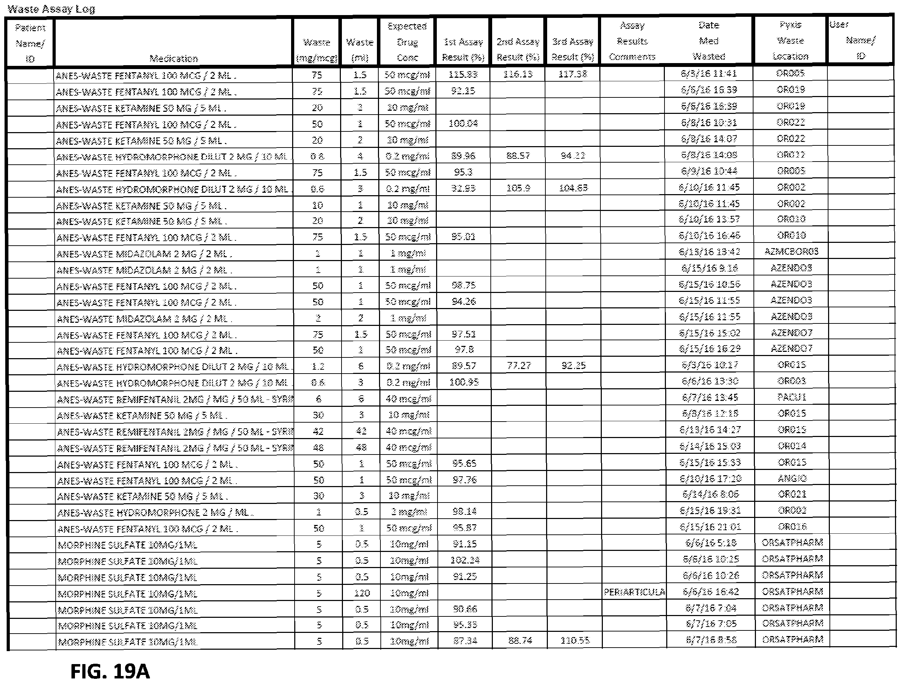

FIG. 19A is an example of a Waste Assay log of the type used with the medication administration auditing system described herein.

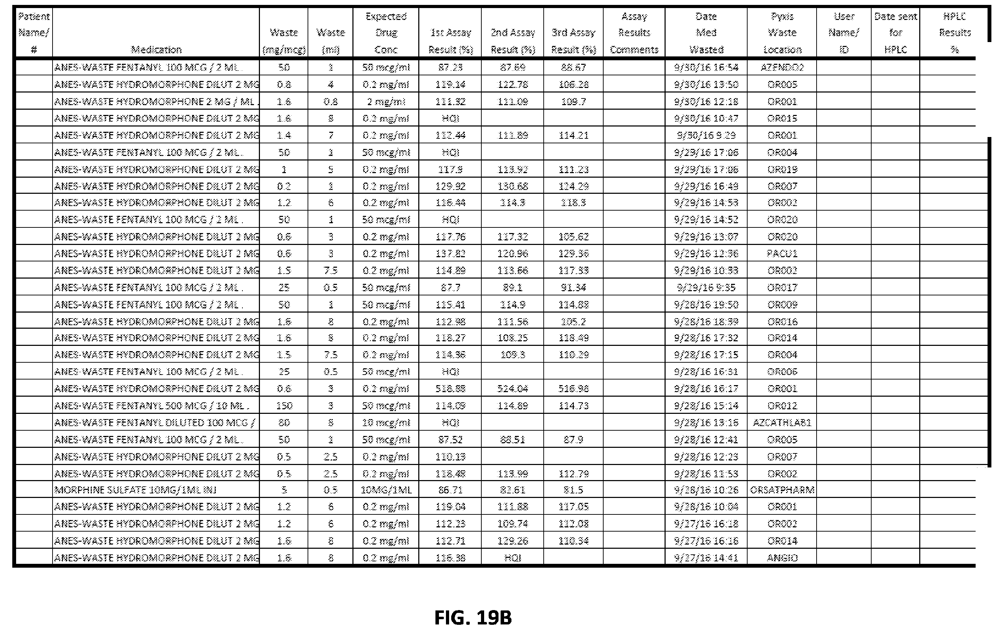

FIG. 19B is an example HPLC (High Performance Liquid Chromatography) log of the type used with the medication administration auditing system described herein.

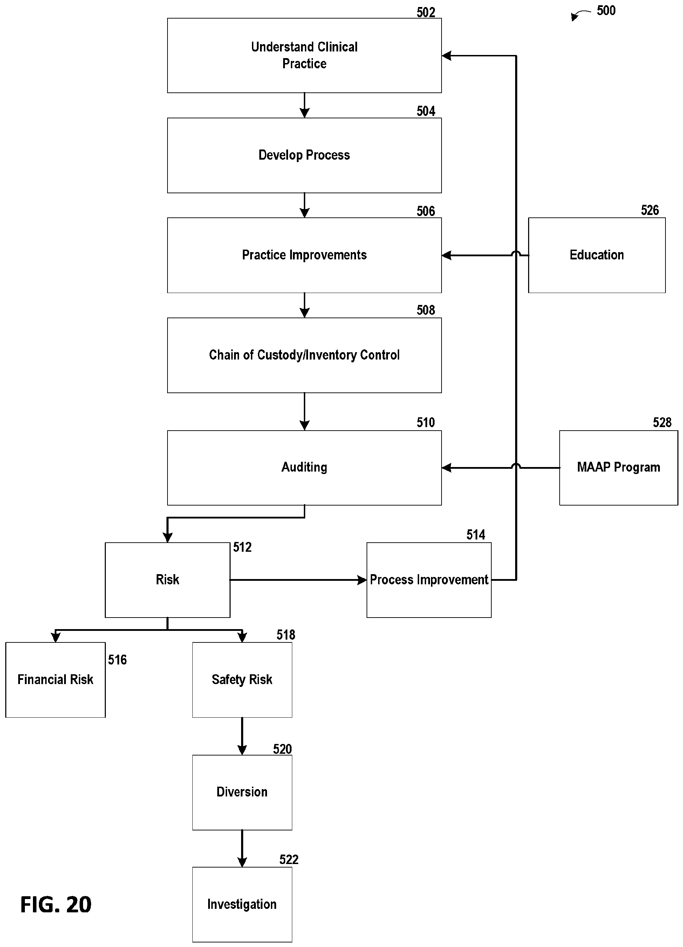

FIG. 20 is a diagram illustrating an example drug diversion prevention model (500), which when implemented in a hospital or other healthcare facility may be used to develop procedures for medication administration auditing in a medical practice area. The model (500) may include gaining an understanding of the clinical practice (502), including personnel types, medications, medication administration processes, practice workflows, etc. Based on this understanding, a process for medication ordering, removal, administration, charting, wasting, and assay may be determined (504), and practice improvements to the process may be identified and implemented (506). The process may be specific to a particular area (such as nursing, PCA/epidural, anesthesia, pharmacy, procedural areas, etc.) or the process may be applied to multiple clinical practice areas. The diversion prevention practice model further includes education of the relevant personnel regarding the process (526). Chain of custody and inventory control procedures for wasting/assay of wasted medications, including identification of drug diversion prevention team personnel, chain of custody and procedures for assay are also developed (508). The process further includes auditing (510) of AMDS data, electronic health record data, assay data and/or infusion pump data to identify possible drug diversion events. The medication administration auditing module, such as analysis module 134, may perform the auditing functions (528). If variances are identified (512), the system may further determine if they are associated with process issues or errors that do not involve diversion event(s) (514) or if they are associated with a potential drug diversion event (520). An investigation procedure by which potential drug diversions may be further investigated and/or confirmed may also be established (522).

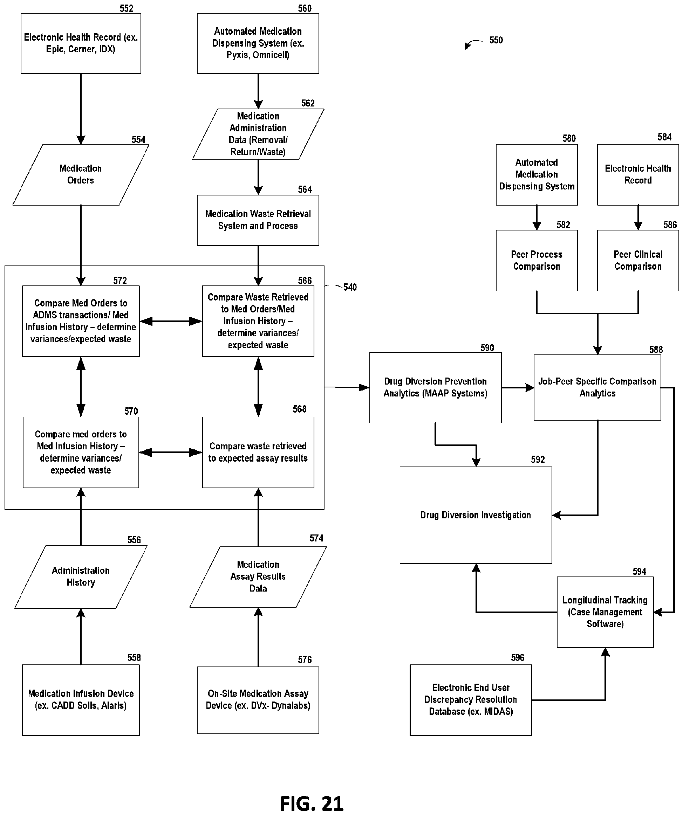

FIG. 21 is another diagram illustrating an example drug diversion prevention model (550). Electronic health record data (552), including medication orders (554), AMDS data (560), including medication administration data from the AMDS (removal/return/waste) (562) as outlined by the medication waste retrieval system and process (564), any medication infusion device data (558), including administration history (556), and the medication assay results data (574) (such as data obtained using spectrographic or High Performance Liquid Chromatography (HPLC) assay techniques), may be analyzed by the medication administration auditing system (such as system 130 of FIG. 1) to identify variances (540). This process may include, for example, comparing medication orders, ADMS transactions, and/or infusion pump history to determine expected waste amounts, and further to compare the actual waste amounts determined by assay with the expected waste amounts (566) (568) (570) (572).

The variances may be further analyzed by the medication administration auditing system to identify potential drug diversion events (590). This may include job-peer specific analysis (582, 586, 588) to "normalize" the data and ensure that users are compared with other users likely to have similar medication administration usage patterns. Once a potential drug diversion has been identified, an investigation may be undertaken to confirm whether drug diversion has occurred (592). Longitudinal tracking (594) of variances over time (596) may be further analyzed to identify potential drug diversions that might be missed if only single data points are reviewed.

The longitudinal tracking and analysis of medication administration auditing information (590) may include review of data by user, employee type or job role, medication, dispensing location, patient, timeframe, practice area/department, geographic location of the healthcare facility, etc. Long term tracking may also allow feedback for education and practice improvement when auditing reveals issues that are not instances of drug diversion.

An understanding of each user's job role and their expected medication use is needed to establish an appropriate baseline (582, 586, 588). By tracking medication transaction history for multiple user's over time, the medication administration auditing system described herein may establish a baseline for each job role (e.g., nurse, physician, etc.) for each criterion (e.g., practice area/department, geographic location, dispensing location, patient, timeframe, etc.) to be reviewed. A user's medication transaction activity (e.g., removal and waste), both individually and over time, may then be compared against the appropriate baseline to identify potential diversion activity (582, 586, 588). Job role, department and location, for example, are considered because medication use and waste can differ greatly by the location and type of service provided by a user. Since a user may work more shifts and/or see more patients, the system may allow for variance from the baseline in certain circumstances.

For example, the system may analyze the number of variances vs. their peer group in the same job role for an expected criterion. In other words, the system may compare a number of variances of a user to a peer group baseline previously established for user's in the same job role. High variance patterns by a particular medication or location may indicate potential diversion. Diverters often have a preferred medication, location and method. Analysis of a user's medication use and waste over time may help to identify these details and find potential diversion. Review of an individual (investigation) may start when behavior outside the norm is identified with all other criteria being as equal as possible.

Variance thresholds may vary across job roles. In addition, one or more of the criteria may be weighted and combined in different ways for different job roles. Once a baseline is built for a criterion, every user that exceeds the criteria baseline can be compared and analyzed. If a user exceeds the average user by more than the permitted variance, a potential diversion may be identified and they may be more deeply reviewed. In another example, if a user has more than three variances in a quarter, a potential diversion may be identified and an investigation started.

The first step during an investigation may be to meet with the individual and discuss the variances. The brief discussion may allow supervisors/administrators to be proactive with practice improvement, education and diversion prevention. If, after a first potential diversion is identified, the individual is diverting and tries to switch methods, the system may be able to identify diversion activity on one of the other criteria being monitored.

For example, to compare an individual Certified Registered Nurse Anesthetist (CRNA) working in Endoscopy to their peers, one or more of the following criteria may be considered:
- How many times did they work in Endoscopy?
- How many patients did they care for?
- What medications did they use?
- What volumes of medication did they use?
- How many waste transactions?
- How many cancels?
- Use of other "meds" (anesthetic gases, propofol, etc. - these can be used to hide indicators (pain scores from a patient) that med was never given.
- Where were the meds removed/wasted? Diverters tend to use the most secluded machines to pull medications.
- Timing of removal of medications - what is the time frame from removal to administration? More time gives more chance to divert/tamper. Crossing transactions between patients is a common method to corrupt monitoring data.
- Waste assay results
- Variances of administration to the net AMDS removal
- Clinical variances vs. expected clinical outcome (example: expect to see a blood pressure drop after administration of hydromorphone injection).

It shall be understood that additional criteria may also be considered, and that these criteria may also be considered for other job roles, and that the disclosure is not limited in this respect.

In this comparison methodology, the medication administration auditing system compares peers in a similar job role. In the CRNA example above, the user will likely work in other areas and that information, created on different criteria, can be used in conjunction to evaluate the user. By comparing users with baselines established for users having the same or similar job role, meaningful comparisons to identify diversion can be made.

Longitudinal tracking and analysis of variances may include review over a number of timeframes (ex. monthly, quarterly, biannually) because diversion may be easily missed due to the timeframe reviewed.

The medication administration auditing system may sort the data by a specific parameter (e.g., user, medication, job role, location, etc.) for further analysis. Electronic data allows for trend analysis of any of the parameters over any timeframe.

In some cases, diversion can be very difficult to identify. The diverter may be expert at employing methods to prevent detection. They may employ multiple types of diversion activities and spread them over an extended period of time such that even if a variance is detected every so often, these variances may not be consistent. In addition, a diverter may be able to explain away a single seemingly random variance as they normally have their explanations prepared in advance. Identification of multiple different types of variances over time, when taken together, may help identify potential diversion activity. For example, the following detected variances may result in identification of a potential diversion:
- High fentanyl use vs. peers - for example, if a user works in Endoscopy where fentanyl can be used in large volumes, volume comparison vs. other users in the department may help demonstrate potential diversion
- High use of anesthetic gases and propofol particularly at the end of the case
- Use of AMDS machines other than where assigned
- Timing of med removals, wastes
- Administration vs. net AMDS removal variances
- Crossing transactions between patients
- Removal of medication on patients that were never assigned
- Assay variances
- Trend analysis of all the items above vs. peers over multiple time frames, including preference for certain days of the week to divert.

Individually, each criterion above, by itself, may not be enough to identify potential diversion. However, the combination of data points, in other words, identification of multiple variances and multiple different types of variances, that may help to identify diversion activity.

In another example, if diversion is suspected, investigators may speak to the individual about certain identified variances with the intent of producing a change in diversion behavior. For example, upon being informed that certain of their diversion activities have been identified, the individual may change from weekly to monthly diversion, change to a different AMDS from which the diverted meds are removed and/or wasted, change diverted medications, etc. The change in behavior may be readily apparent when trend analyzed and may help to confirm if real diversion activity exists.

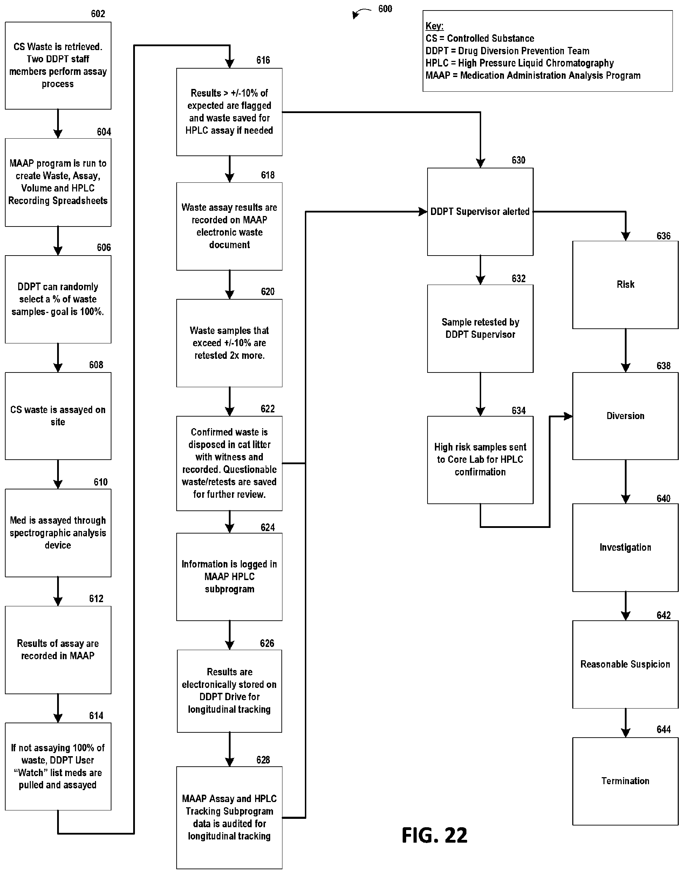

FIG. 22 is a flowchart illustrating an example waste assay tracking process (600). This includes the procedures to be followed by a drug diversion prevention team (DDPT) for retrieval and assay of the medical waste (also referred to as "controlled substance" or "CS" waste). The waste assay tracking process also establishes a chain of custody to maintain the integrity of the medical waste through the retrieval and assay procedures.

CS waste is retrieved (such as from an AMDS) (602). Two DDPT members are assigned to perform an appropriate assay process on the retrieved waste. The assay may vary depending upon the type of controlled substance. Documentation (e.g., spreadsheets or other appropriate documentation) are created to record data generated during the assay process

(604). The DDPT may randomly test a pre-defined percentage (e.g., 50%) of all CS waste; however, in some examples, 100% of the medical waste is tested to increase the likelihood that all diversion events will be detected (606). As another example, if the procedures do not call for assay of 100% of the CS waste, the DDPT may establish a "watch list" of high risk medications that are always assayed (614), and assay a pre-defined percentage of medications that are not on the watch list.

The CS waste is assayed (608) by the assigned members of the DDPT. In some examples, the assay takes place on site to help maintain the chain of custody and thus the integrity of the CS waste. As mentioned above, the assay may vary depending upon the type of CS waste being tested. In some examples, the assay may include a determination of the volume of the CS waste and /or a spectrographic analysis of the CS waste to determine its chemical composition (610). The results are recorded (612) (e.g., in auditing data 146 shown in FIG. 1, for example).

As described herein, based on the AMDS data (indicating what and how much of a medication was dispensed, and also indicating how much of the dispensed medication was wasted/returned, if any) and the electronic health record data (indicating how much of the dispensed medication was administered to the patient), the medication administration auditing system determines the expected volume and concentration of the retrieved CS waste. If the results of the assay are not within a defined percentage of the expected volume and concentration (e.g., 10% or other appropriate percentage) the results are flagged and the CS waste is sent for further analysis (616). The results of the assay and the comparison with expected are also recorded (such as in auditing data 146 shown in FIG. 1, for example) (618).

In the example of FIG. 22, any CS waste not within the defined percentage of expected volume and concentration are tested an additional 2 times to confirm the results of the initial assay (620). CS waste that passes the initial assay are disposed of and witnessed by a DDPT member (622). Questionable CS waste that does not meet the pre-defined tolerances with expected are saved for further review (622). Further review may include analysis using high performance liquid chromatography (HPLC) (624) or other alternative assay techniques. The results of all assays are stored for longitudinal tracking (626, 628). If a first assay does not fall within the pre-defined tolerance of expected, a DDPT supervisor or another team member may be alerted (630). The sample may then be tested again by the supervisory DDPT member (632). Again, this step may be implemented to further confirm the results of the initial assay and to increase confidence in the results for all persons involved. As mentioned above, further review may include analysis using high performance liquid chromatography (HPLC) (634) or other alternative assay techniques.

If a potential diversion is confirmed by these additional assay procedures (638) and/or by the longitudinal tracking, an investigation may be conducted (640) to identify the persons involved and determine the circumstances of the diversion(s). If the investigation concludes with a reasonable suspicion that one or more employees have been diverting (642), repercussions may include termination of the employee(s) (644).

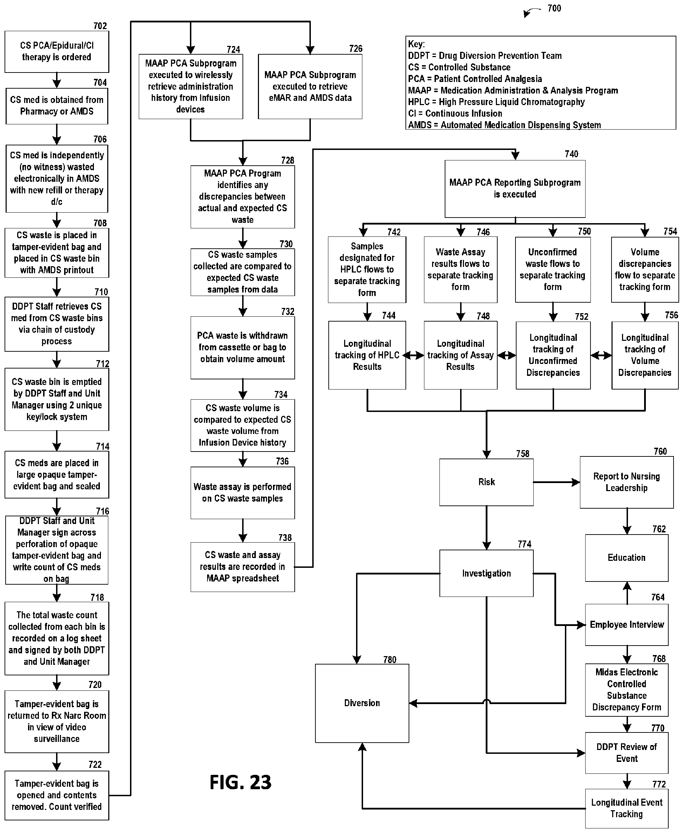

FIG. 23 is a flow chart illustrating an example drug diversion prevention and identification auditing process (700). This process will be described with respect to a patient controlled analgesia (PCA), epidural, or continuous infusion practice area of a healthcare facility; however, it shall be understood that some or all the process steps may be used for other areas within a hospital or healthcare facility, and that the disclosure is not limited in this respect. This includes the procedures to be followed by a drug diversion prevention team (DDPT) for retrieval and assay of the medical waste (also referred to as "controlled substance" or "CS" waste). The process includes a waste assay tracking process that establishes a chain of custody to maintain the integrity of the medical waste through the retrieval and assay procedures.

When a CS PCA/Epidural/CI therapy is ordered (702), a user executes an AMDS transaction or pharmacy transaction to obtain the CS medication (704). Once the CS medication is administered to the patient, the CS medication may be independently wasted (no witness required) via an AMDS (706) and results electronically stored by the AMDS as described herein. Wasting of the CS medication may include placing the CS waste in a tamper-evident bag and placing the bag in the CS waste bin associated with the AMDS (708). The waste retrieval process may include retrieval by a DDPT member or members according to the defined chain of custody procedures (710). In this example, the CS waste bin is emptied by DDPT staff and unit manger using 2 unique keylock system (712). The CS meds are placed in an opaque tamper evident bag and sealed (714). The DDPT staff and unit manager sign across a perforation of the tamper-evident bag and write a count of the CS meds on the bag (716). A total waste count collected from each AMDS waste bin is recorded on a log sheet and signed by both the DDPT staff and unit manager (718). The tamper-evident bag is returned to an area or room assigned for assay in view of video surveillance (720). The tamper-evident bag is opened, the contents are removed, and the counts are verified (722).

The medication administration auditing system receives administration histories (either directly or indirectly) from infusion devices (such as infusion devices 162 of FIG. 1) (724). This data includes the amount of CS medication administered to a patient by the infusion device. The medication administration auditing system also receives the electronic health record and AMDS data regarding the CS medication (726). This data includes the charted amount of the CS medication administered to the patient and the amount of the CS medication dispensed by/wasted to the AMDS, respectively. The medication administration auditing system identifies any discrepancies between the actual amount of CS wasted back into the AMDS and the expected CS waste (728). To do this, an assay procedure is performed, including collecting CS waste samples (730), determining the volume of the CS waste sample (732), and comparing the CS waste volume to the expected CS waste volume (the expected waste volume determined with reference to the administration data from the infusion device) (734). In addition, an assay is performed on the CS waste samples to identify the chemical composition of the wasted material (736). This is further compared to the expected CS waste (738). The results of the assay are stored for analysis (738).

The medication administration auditing system may further break down the results of the assay procedures into four subgroups (740). Samples not meeting the pre-defined tolerances of the expected CS waste may be designated for further assay (such as additional spectrographic or HPLC analysis) (742). The further assays are performed, the results stored, and longitudinal tracking and analysis is performed on those samples to identify potential diversions (742,744). Similarly, confirmed waste assay results are also separately tracked and analyzed over time for comparison purposes (746, 748). Unconfirmed CS waste (e.g., CS waste not satisfying the predefined margin for error) are separately tracked and analyzed over time (750, 752) as are volume discrepancies (754, 756). One or more discrepancies may rise to the level of a risk that diversion is occurring (758), at which point an investigation may be conducted (774) to confirm whether diversion has occurred (780). The investigation may include a report to nursing or other appropriate leadership (760), an employee interview (764), a DDPT review (770), and further longitudinal tracking and analysis (772). An electronic controlled substance discrepancy form may be maintained in an appropriate database (768). Education may be provided for relevant employees in the event of errors that do not involve diversion (762).

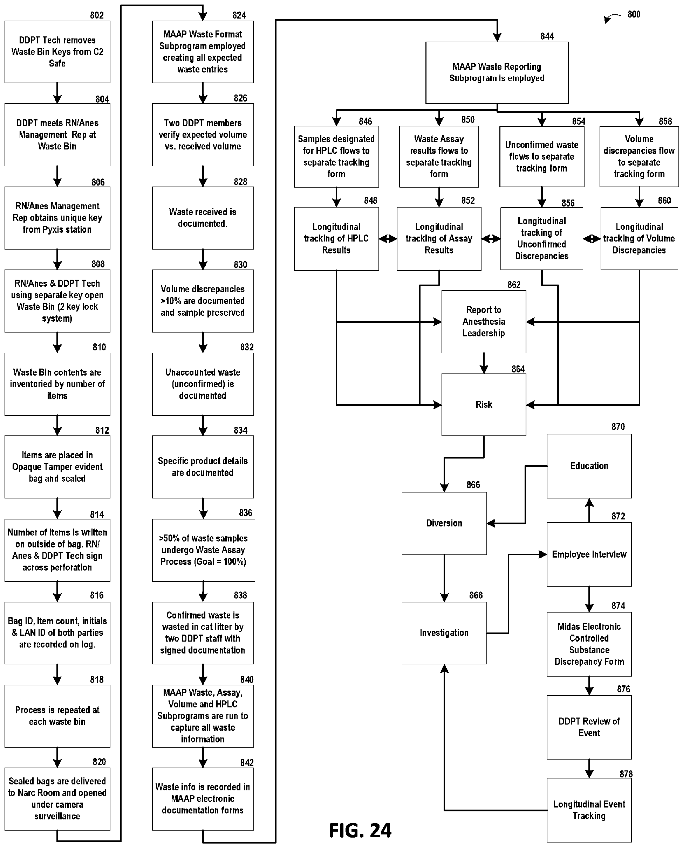

FIG. 24 is a flow chart illustrating another example drug diversion prevention and identification auditing process (800). This process will be described with respect to an anesthesia practice area of a healthcare facility; however, it shall be understood that some or of all the process steps may be used for other areas within a hospital or healthcare facility, and that the disclosure is not limited in this respect. This includes the procedures to be followed by a drug diversion prevention team (DDPT) for retrieval and assay of the CS waste. The retrieval process is shown as being specific to a Pyxis^{™} automated medication dispensing system, and CS waste bins having a two-key lock system; however, it shall be understood that any automated mediation dispensing system may be used, and that other secured waste bins may be used, and that the disclosure is not limited in this respect. The process includes a waste assay tracking process that establishes a secure chain of custody to maintain the integrity of the medical waste through the retrieval and assay procedures.

The waste retrieval process begins when a first person, such as a DDPT member removes a first waste bin key from a secured location, such as a safe (802). The DDPT member meets a second person, such as an RN or anesthesia management staff member at the AMDS waste bin (804). The second person obtains a second waste bin key for the AMDS station (806). The first and second persons open the waste bin using the 2-key lock system (808). The waste bin contents are inventoried by number of items (810). The CS waste items are placed in an opaque tamper-evident bag and sealed (812). The number of CS waste items is written on the outside of the bag, and the first and second persons each sign/initial the bag across a perforation (814). The bag ID, item count, initials of the first and second persons are recorded on a log (816). This process (802-816) is repeated at each waste bin (818). The sealed CS waste bags are delivered to an area or room designated for CS waste assay under video surveillance (820).

During the CS waste assay process, an electronic record is created for each item of expected CS waste (824). Two DDPT members verify the expected volume of CS waste with the received volume of CS waste (826), and this data is electronically documented (828). Volume discrepancies greater than a predetermined threshold (e.g., 10%) are documented and the CS waste sample is preserved for future testing (830, 832, 834). A predetermined percentage (e.g., at least 50%) of CS waste samples are assayed (836). CS waste that is assayed within the acceptable limits is properly disposed of by two DDPT staff with signed documentation (838). All the CS waste data generated during the assay process is recorded electronically, such as in auditing data 146 of FIG. 1 (840, 842).

The medication administration auditing system may further break down the results of the assay procedures into four subgroups (844). Samples not meeting the pre-defined tolerances of the expected CS waste may be designated for further assay (such as additional spectrographic or HPLC analysis) (846). The further assays are performed, the results stored, and longitudinal tracking and analysis is performed on those samples to identify potential diversions (846, 848). Similarly, confirmed waste assay results are also separately tracked and analyzed over time for comparison purposes (850, 852). Unconfirmed CS waste (e.g., CS waste not satisfying the predefined margin for error) are separately tracked and analyzed over time (854, 856) as are volume discrepancies (858, 860). One or more discrepancies may identify a risk that diversion is occurring (864). Anesthesia leadership may be notified (862), and an investigation may be conducted (868) to confirm whether diversion has occurred (866). The investigation may include a report to anesthesia or other appropriate leadership (862), an employee interview (872), a DDPT review (876), and further longitudinal tracking and analysis (878). An electronic controlled substance discrepancy form may be maintained in an appropriate database (874). Education may be provided for relevant employees in the event of errors that do not involve diversion (870).

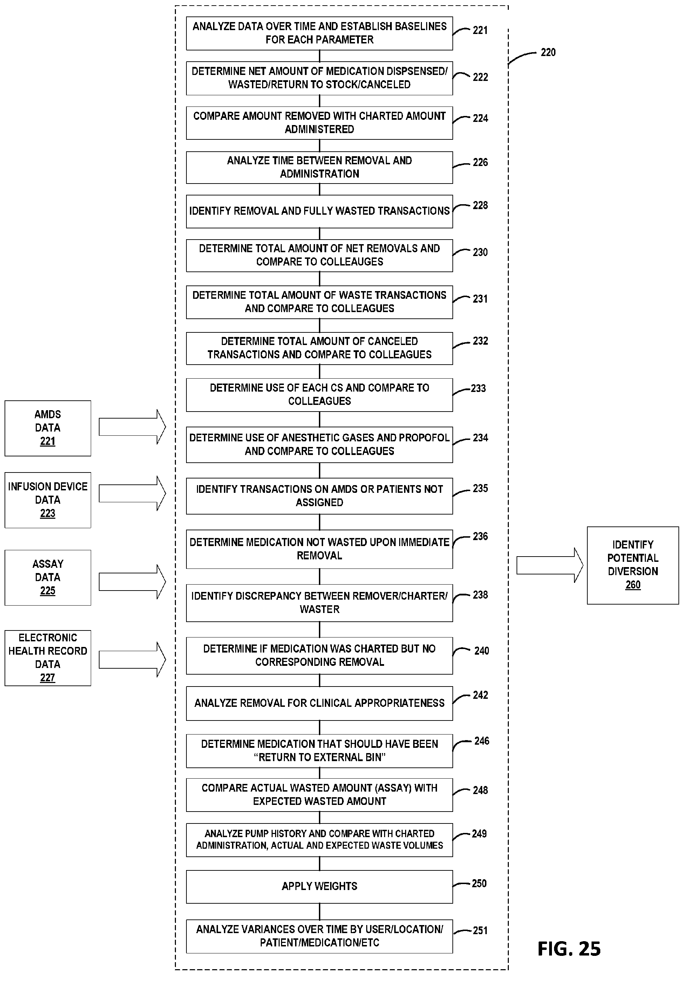

FIG. 25 is a diagram illustrating an example overall process (220) executed by a medication administration auditing system, such as medication administration auditing system 130 shown in FIG. 1. The medication administration auditing system (such as medication administration auditing system 130 of FIG. 1) receives AMDS transaction data (including controlled substance medication dispense data and waste data), infusion device data (including data regarding administration of controlled substance medications by the infusion device), assay data (including results of any controlled substance waste assays), and electronic health record data (including charted medications prescribed and administered). The medication administration auditing system executes an auditing/analysis program 220 (such as analysis application module 134 of FIG. 1) to identify potential drug diversion events.

The auditing/analysis 220 may include one or more of the functions shown in FIG. 25. The system may, for example, receive and analyze AMDS data 221, infusion device data 223, assay data 225, and electronic health record data 227, either individually or in any combination, over time to establish baselines corresponding to each peer group, and for each parameter/criteria analyzed (medication, timeframe, geographic location, etc.) (221).

The system may further determine, from AMDS transaction data, a net amount of medication dispensed within a defined period of time, a net amount of medication wasted within a defined period of time, a net amount of medication returned to stock within a defined period of time, and/or a net number of transactions canceled within a defined period of time (222). The system may further compare an amount of a medication removed/dispensed from an AMDS with a charted amount of the medication administered to a patient (224). The system may further analyze the time frame between a medication dispense and administration of the medication to a patient (226). The system may further identify transaction in which a medication was removed/dispensed by an AMDS and the entire amount of the medication was fully wasted (228).

The system may further determine the net number of dispense/removals by a user within a defined period of time and compare it with a defined peer group (230). The system may further determine the total amount of waste transactions by a user within a defined period of time and compare it with a defined peer group (231). The system may further determine the total number of canceled transactions by a user within a defined period of time and compare it with a defined peer group (232). The system may further determine the amount/volume of each controlled substance used by a user within a defined period of time and compare it with a defined peer group (233). The system may determine the amount/volume of anesthetic gases and propofol within a defined period of time and compare it with a defined peer group (234).

The system may further identify transactions by a user on AMDS machines or recorded against patients not assigned to a user (235). The system may further determine medication that was not wasted upon immediate removal (236). The system may further identify any discrepancies between the user who removes a medication from an AMDS, the person who charts the medication, and the person who wastes the medication (238). The system may further determine if a medication was charted but has no corresponding removal from an AMDS (was never dispensed) (240). The system may further analyze any dispense/removal transactions with clinical appropriateness (242). The system may further identify any medications that should have been "returned to external bin" (246).

The system may further compare an actual amount (e.g., volume or weight) wasted as determined by assay with an expected amount wasted (248). The system may further analyze an infusion device history and compare an actual amount of a medication administered to a patient with a charted amount of the medication to be administered and with an expected waste amount of the medication (249).

The system may further apply weights to one or more of the criteria, and/or combine the criteria in different ways for different job roles (250). The system may further analyze the AMDS data, the infusion device data, assay data, and electronic health record data over time (251). Detection of one or more variances during analysis (220) performed by the medication administration auditing system may identify potential diversion activity (260).

The techniques described in this disclosure, including functions performed by a processor, controller, control unit, or control system, may be implemented within one or more of a general purpose microprocessor, digital signal processor (DSP), application specific integrated circuit (ASIC), field programmable gate array (FPGA), programmable logic devices (PLDs), or other equivalent logic devices. Accordingly, the terms "processor" "processing unit" or "controller," as used herein, may refer to any one or more of the foregoing structures or any other structure suitable for implementation of the techniques described herein.

The various components illustrated herein may be realized by any suitable combination of hardware, firmware, and/or software. In the figures, various components are depicted as separate units or modules. However, all or several of the various components described with reference to these figures may be integrated into combined units or modules within common hardware, firmware, and/or software. Accordingly, the representation of features as components, units or modules is intended to highlight particular functional features for ease of illustration, and does not necessarily require realization of such features by separate hardware, firmware, or software components. In some cases, various units may be implemented as programmable processes performed by one or more processors or controllers.

Any features described herein as modules, devices, or components may be implemented together in an integrated logic device or separately as discrete but interoperable logic devices. In various aspects, such components may be formed at least in part as one or more integrated circuit devices, which may be referred to collectively as an integrated circuit device, such as an integrated circuit chip or chipset. Such circuitry may be provided in a single integrated circuit chip device or in multiple, interoperable integrated circuit chip devices, and may be used in any of a variety of applications and devices.

If implemented in part by software, the techniques may be realized at least in part by a computer-readable data storage medium comprising code with instructions that, when executed by one or more processors or controllers, performs one or more of the methods described in this disclosure. The computer-readable storage medium may form part of a computer program product, which may include packaging materials. The computer-readable medium may comprise random access memory (RAM) such as synchronous dynamic random access memory (SDRAM), read-only memory (ROM), non-volatile random access memory (NVRAM), electrically erasable programmable read-only memory (EEPROM), embedded dynamic random access memory (eDRAM), static random access memory (SRAM), flash memory, magnetic or optical data storage media. Any software that is utilized may be executed by one or more processors, such as one or more DSP's, general purpose microprocessors, ASIC's, FPGA's, or other equivalent integrated or discrete logic circuitry.

The scope of protection is defined by the annexed claims.

## Claims

1. A computer-implemented method (134, 136) for auditing of medication administration in an automated medication dispensing system (100), the method comprising:
receiving medication dispense transaction data associated with the automated medication dispensing system (104), the medication dispense transaction data including at least a user identifier, a medication identifier, a patient identifier, and a dispensed amount of a medication associated with the medication identifier;
receiving electronic health record data corresponding to the medication dispense transaction data, the electronic health record data including at least a patient identifier, a prescribed medication, and an administered amount of the medication;
comparing the medication dispense transaction data to the corresponding electronic health record data;
determining an expected wasted amount of the medication based on the dispensed amount of the medication and the administered amount of the medication;
receiving assay data corresponding to a quantitative assay indicative of an actual wasted amount of the medication;
receiving medication waste transaction data associated with the automated medication dispensing system, the received medication waste transaction data being indicative of a wasted amount of the medication;
comparing the dispensed amount of the medication, the wasted amount of the medication, the administered amount of the medication, the expected wasted amount of the medication and the actual wasted amount of the medication associated with a user with dispensed amounts of the medication, wasted amounts of the medication, administered amounts of the medication, expected wasted amounts of the medication and actual wasted amounts of the medication associated with one or more peers of the user in a similar job role;
identifying the variance indicative of a potential drug diversion event based on the comparison; and
generating one or more reports including at least one of the results of the comparison and the identified variance and including results of the analysis of the dispensed amount of medication and/or the wasted amount of medication over time based on at least one of user, medication, type of user, location, timeframe, practice, wherein results of the analysis are for improvement of patient safety.

2. The method of claim 1, wherein the medication dispense transaction data comprises medication waste transaction data or medication return to stock transaction data.

3. The method of claim 1 further comprising receiving assay data corresponding to a quantitative assay for a predetermined percentage of waste transactions.

4. The method of claim 1 further comprising analyzing the medication dispense transaction data over a period of time to identify trends in the transaction data.

5. The method of claim 1 further comprising analyzing at least a portion of the medication dispense transaction data that is associated with a specific health care provider.

6. The method of claim 1, further comprising:
receiving infusion medication administration data indicative of an actual amount of an infusion medication administered by an infusion device;
receiving assay data corresponding to a quantitative assay indicative of an actual wasted amount of the infusion medication;
determining an expected wasted amount of the infusion medication based on a dispensed amount of the infusion medication and the administered amount of the medication; and
identifying the variance indicative of the potential drug diversion event based on the dispensed amount of the infusion medication, the administered amount of the infusion medication, the wasted amount of the infusion medication, the expected wasted amount of the infusion medication, and the actual wasted amount of the infusion medication.

7. A medication administration auditing system (100), comprising: at least one processor (132) configured to:
receive medication dispense transaction data associated with a plurality of dispense transactions performed in an automated medication dispensing system (104), the medication
dispense transaction data including, for each of the plurality of dispense transactions, a user identifier, a medication identifier, a patient identifier, and a dispensed amount of a medication;
receive electronic health record data corresponding to the medication dispense transaction data, the electronic health record data including at least a patient identifier, a prescribed medication, and an administered amount of the medication;
receive medication waste transaction data associated with a plurality of waste transactions performed in an automated medication dispensing system, the medication waste transaction data including, for each of the plurality of waste transactions, a user identifier, a medication identifier, a patient identifier, and a wasted amount of a medication;
for each of the plurality of dispense transactions, compare the medication dispense transaction data to the corresponding electronic health record data;
for each of the plurality of dispense transactions, determine an expected wasted amount of the medication based on the dispensed amount of the medication and the administered amount of the medication;
for each of the plurality of waste transactions, receive assay data corresponding to a quantitative assay indicative of an actual wasted amount of the medication;
compare the dispensed amount of the medication, the wasted amount of the medication, the administered amount of the medication, the expected wasted amount of the medication and the actual wasted amount of the medication associated with a user with dispensed amounts of the medication, wasted amounts of the medication, administered amounts of the medication, expected wasted amounts of the medication and actual wasted amounts of the medication associated with one or more peers of the user in a similar job role;
identify the variance indicative of the potential drug diversion event based on the comparison, and
generate one or more reports including at least one of the results of the comparison and the identified variance and including results of the analysis of the dispensed amount of medication and/or the wasted amount of medication over time based on at least one of user, medication, type of user, location, timeframe, practice, wherein results of the analysis are for improvement of patient safety.

8. The medication administration auditing system of claim 7, wherein the at least one processor is further configured to:
for each of a plurality of user identifiers, compare a number of identified variances with a number of identified variances associated with a peer group baseline corresponding to the user.

9. The medication administration auditing system of claim 7, wherein the at least one processor is further configured to:
for each of a plurality of user identifiers and for each of the plurality of dispense transactions associated with the respective user identifier, compare the dispensed amount of the medication with an average dispensed amount of the medication associated with a peer group baseline corresponding to the user.

10. The medication administration auditing system of claim 7, wherein the at least one processor is further configured to:
for each of the plurality of waste transactions, receive assay data corresponding to a quantitative assay indicative of an actual wasted amount of the medication; and
for each of a plurality of user identifiers and for each of the plurality of waste transactions, compare the actual wasted amount of the medication with an average wasted amount of the medication associated with a peer group baseline corresponding to the user

11. The medication administration auditing system of claim 7, wherein the at least one processor is further configured to:
receive infusion medication administration data associated with a plurality of infusion devices, the infusion medication administration data including, for each administration of an infusion medication by the plurality of infusion devices, a user identifier, an infusion medication identifier, a patient identifier, and an actual administered amount of the infusion medication;
receive infusion medication waste transaction data associated with a plurality of infusion medication waste transactions performed in an automated medication dispensing system, the infusion medication waste transaction data including, for each of the plurality of infusion medication waste transactions, a user identifier, an infusion medication identifier, a patient identifier, and a wasted amount of an infusion medication;
for each administration of an infusion medication by the plurality of infusion devices, compare the administered amount of the medication with a third peer group baseline corresponding to the user identifier;
for each of the plurality of waste transactions, compare the wasted amount of the medication with a fourth peer group baseline corresponding to the user identifier; and
identify a variance indicative of a potential drug diversion for each one of the comparisons that satisfy a variance threshold.

## Patentansprüche

1. Computerimplementiertes Verfahren (134, 136) zum Auditieren einer Medikamentenverabreichung in einem automatisierten Medikamentenabgabesystem (100), wobei das Verfahren umfasst:
Erhalten von Medikamentenabgabe-Transaktionsdaten, die dem automatisierten Medikamentenabgabesystem (104) zugewiesen sind,
wobei die Medikamentenabgabe-Transaktionsdaten zumindest eine Benutzerkennung, eine Medikamentenkennung, eine Patientenkennung, und eine abgegebene Menge eines Medikaments, das der Medikamentenkennung zugewiesen ist, umfassen;
Erhalten von elektronischen Patientenaktendaten entsprechend den Medikamentenabgabe-Transaktionsdaten, wobei die elektronischen Patientenaktendaten zumindest eine Patientenkennung, ein verordnetes Medikament, und
eine verabreichte Menge des Medikaments umfassen;
Vergleichen der Medikamentenabgabe-Transaktionsdaten mit den entsprechenden elektronischen Patientenaktendaten;
Bestimmen einer erwarteten Abfallmenge des Medikaments basierend auf der abgegebenen Menge des Medikaments und der verabreichten Menge des Medikaments;
Erhalten von Untersuchungsdaten entsprechend einer quantitativen Untersuchung, die eine tatsächliche Abfallmenge des Medikaments angeben;
Erhalten von Medikamentenabfall-Transaktionsdaten, die dem automatisierten Medikamentenabgabesystem zugewiesen sind, wobei die erhaltenen Medikamentenabfall-Transaktionsdaten eine Abfallmenge des Medikaments angeben;
Vergleichen der abgegebenen Menge des Medikaments, der Abfallmenge des Medikaments, der verabreichten Menge des Medikaments, der erwarteten Abfallmenge des Medikaments und der tatsächlichen Abfallmenge des Medikaments, die einem Benutzer zugewiesen sind, mit abgegebenen Mengen des Medikaments, Abfallmengen des Medikaments, verabreichten Mengen des Medikaments, erwarteten Abfallmengen des Medikaments und tatsächlichen Abfallmengen des Medikaments, die einem oder mehreren Peers des Benutzers in einer ähnlichen Arbeitsrolle zugewiesen sind;
Identifizieren der Abweichung, die auf ein potenzielles Ereignis einer Abzweigung von Arzneimitteln hinweist, basierend auf dem Vergleich; und
Erstellen eines oder mehrerer Berichte, die mindestens eines aus den Ergebnissen des Vergleichs und der identifizierten Abweichung umfassen und Ergebnisse der Analyse der abgegebenen Menge des Medikaments und/oder der Abfallmenge des Medikaments im Laufe der Zeit basierend auf mindestens einem aus Benutzer, Medikament, Benutzertyp, Ort, Zeitrahmen, Praxis umfassen, wobei die Ergebnisse der Analyse der Verbesserung der Patientensicherheit dienen.

2. Verfahren nach Anspruch 1, wobei die Medikamentenabgabe-Transaktionsdaten Medikamentenabfall-Transaktionsdaten oder Transaktionsdaten zur Rückkehr eines Medikaments in den Bestand umfassen.

3. Verfahren nach Anspruch 1, das ferner das Erhalten von Untersuchungsdaten entsprechend einer quantitativen Untersuchung für eine vorbestimmte Prozentzahl an Abfalltransaktionen umfasst.

4. Verfahren nach Anspruch 1, das ferner das Analysieren der Medikamentenabgabe-Transaktionsdaten über einen Zeitraum umfasst, um Trends in den Transaktionsdaten zu identifizieren.

5. Verfahren nach Anspruch 1, das ferner das Analysieren von zumindest einem Teil der Medikamentenabgabe-Transaktionsdaten umfasst, die einem bestimmten Gesundheitsdienstleister zugewiesen sind.

6. Verfahren nach Anspruch 6, das ferner umfasst:
Erhalten der Infusionsmedikament-Verabreichungsdaten, die eine tatsächliche Menge eines Infusionsmedikaments angeben, das von einer Infusionsvorrichtung verabreicht wird;
Erhalten von Untersuchungsdaten entsprechend einer quantitativen Untersuchung, die eine tatsächliche Abfallmenge des Infusionsmedikaments angeben;
Bestimmen einer erwarteten Abfallmenge des Infusionsmedikaments basierend auf einer abgegebenen Menge des Infusionsmedikaments und der verabreichten Menge des Medikaments; und
Identifizieren der Abweichung, die auf ein potenzielles Ereignis der Abzweigung von Arzneimitteln hinweist, basierend auf der abgegebenen Menge des Infusionsmedikaments, der verabreichten Menge des Infusionsmedikaments,
der Abfallmenge des Infusionsmedikaments, der erwarteten Abfallmenge des Infusionsmedikaments, und der tatsächlichen Abfallmenge des Infusionsmedikaments.

7. Medikamentenverabreichungs-Auditsystem (100), das aufweist:
mindestens einen Prozessor (132), der für Folgendes ausgebildet ist:
Erhalten von Medikamentenabgabe-Transaktionsdaten, die einer Vielzahl von Abgabetransaktionen zugewiesen sind, die in einem automatisierten Medikamentenabgabesystem (104) durchgeführt werden, wobei die Medikamentenabgabe-Transaktionsdaten für jede der Vielzahl von Abgabetransaktionen eine Benutzerkennung, eine Medikamentenkennung, eine Patientenkennung, und eine abgegebene Menge eines Medikaments umfassen;
Erhalten von elektronischen Patientenaktendaten entsprechend den Medikamentenabgabe-Transaktionsdaten, wobei die elektronischen Patientenaktendaten zumindest eine Patientenkennung, ein verordnetes Medikament, und eine verabreichte Menge des Medikaments umfassen;
Erhalten von Medikamentenabfall-Transaktionsdaten, die einer Vielzahl von Abfalltransaktionen zugewiesen sind, die in einem automatisierten Medikamentenabgabesystem durchgeführt werden, wobei die Medikamentenabfall-Transaktionsdaten für jede der Vielzahl von Abfalltransaktionen eine Benutzerkennung, eine Medikamentenkennung, eine Patientenkennung, und eine Abfallmenge eines Medikaments umfassen;
für jede der Vielzahl von Abgabetransaktionen, Vergleichen der Medikamentenabgabe-Transaktionsdaten mit den entsprechenden elektronischen Patientenaktendaten;
für jede der Vielzahl von Abgabetransaktionen, Bestimmen einer erwarteten Abfallmenge des Medikaments basierend auf der abgegebenen Menge des Medikaments und der verabreichten Menge des Medikaments;
für jede der Vielzahl von Abfalltransaktionen, Erhalten von Untersuchungsdaten entsprechend einer quantitativen Untersuchung, die eine tatsächliche Abfallmenge des Medikaments angeben;
Vergleichen der abgegebenen Menge des Medikaments, der Abfallmenge des Medikaments, der verabreichten Menge des Medikaments, der erwarteten Abfallmenge des Medikaments und der tatsächlichen Abfallmenge des Medikaments, die einem Benutzer zugewiesen sind, mit abgegebenen Mengen des Medikaments, Abfallmengen des Medikaments, verabreichten Mengen des Medikaments, erwarteten Abfallmengen des Medikaments und tatsächlichen Abfallmengen des Medikaments, die einem oder mehreren Peers des Benutzers in einer ähnlichen Arbeitsrolle zugewiesen sind;
Identifizieren der Abweichung, die auf das Ereignis der potenziellen Abzweigung von Arzneimitteln hinweist, basierend auf dem Vergleich; und
Erstellen eines oder mehrerer Berichte, die mindestens eines aus den Ergebnissen des Vergleichs und der identifizierten Abweichung umfassen und Ergebnisse der Analyse der abgegebenen Menge des Medikaments und/oder der Abfallmenge des Medikaments im Laufe der Zeit basierend auf mindestens einem aus Benutzer, Medikament, Benutzertyp, Ort, Zeitrahmen, Praxis umfassen, wobei die Ergebnisse der Analyse der Verbesserung der Patientensicherheit dienen.

8. Medikamentenverabreichungs-Auditsystem nach Anspruch 7, wobei der mindestens eine Prozessor ferner für Folgendes ausgebildet ist:
für jede einer Vielzahl von Benutzerkennungen, Vergleichen einer Anzahl von identifizierten Abweichungen mit einer Anzahl von identifizierten Abweichungen, die einem dem Benutzer entsprechenden Peer-Group-Basiswert zugewiesen sind.

9. Medikamentenverabreichungs-Auditsystem nach Anspruch 7, wobei der mindestens eine Prozessor ferner für Folgendes ausgebildet ist:
für jede einer Vielzahl von Benutzerkennung und für jede der Vielzahl von Abgabetransaktionen, die der jeweiligen Benutzerkennung zugewiesen sind,
Vergleichen der abgegebenen Menge des Medikaments mit einer durchschnittlich abgegebenen Menge des Medikaments, die einem dem Benutzer entsprechenden Peer-Group-Basiswert zugewiesen ist.

10. Medikamentenverabreichungs-Auditsystem nach Anspruch 7, wobei der mindestens eine Prozessor ferner für Folgendes ausgebildet ist:
für jede der Vielzahl von Abfalltransaktionen, Erhalten von Untersuchungsdaten entsprechend einer quantitativen Untersuchung, die eine tatsächliche Abfallmenge des Medikaments angeben; und
für jede einer Vielzahl von Benutzerkennungen und für jede der Vielzahl von Abfalltransaktionen, Vergleichen der tatsächlichen Abfallmenge des Medikaments mit einer durchschnittlichen Abfallmenge des Medikaments, die einem dem Benutzer entsprechenden Peer-Group-Basiswert zugewiesen ist.

11. Medikamentenverabreichungs-Auditsystem nach Anspruch 7, wobei der mindestens eine Prozessor ferner für Folgendes ausgebildet ist:
Erhalten von Infusionsmedikament-Verabreichungsdaten, die einer Vielzahl von Infusionsvorrichtungen zugewiesen sind, wobei die Infusionsmedikament-Verabreichungsdaten für jede Verabreichung eines Infusionsmedikaments durch die Vielzahl von Infusionsvorrichtungen eine Benutzerkennung, eine Infusionsmedikamentenkennung, eine Patientenkennung, und eine tatsächliche verabreichte Menge des Infusionsmedikaments umfassen;
Erhalten von Infusionsmedikament-Abfalltransaktionsdaten, die einer Vielzahl von Infusionsmedikament-Abfalltransaktionen zugewiesen sind, die in einem automatisierten Medikamentenabgabesystem durchgeführt werden, wobei die Infusionsmedikament-Abfalltransaktionsdaten für jede der Vielzahl von Infusionsmedikament-Abfalltransaktionen eine Benutzerkennung, eine Infusionsmedikamentenkennung, eine Patientenkennung, und eine Abfallmenge eines Infusionsmedikaments umfassen;
für jede Verabreichung eines Infusionsmedikaments durch die Vielzahl von Infusionsvorrichtungen, Vergleichen der verabreichten Menge des Medikaments mit einem der Benutzerkennung entsprechenden dritten Peer-Group-Basiswert;
für jede der Vielzahl von Abfalltransaktionen, Vergleichen der Abfallmenge des Medikaments mit einem der Benutzerkennung entsprechenden vierten Peer-Group-Basiswert; und
Identifizieren einer Abweichung, die auf eine potenzielle Abzweigung von Arzneimitteln hinweist, für jeden der Vergleiche, die einen Abweichungsschwellenwert erfüllen.

## Revendications

1. Procédé (134, 136) mis en œuvre par ordinateur pour l'audit de l'administration de médicaments dans un système automatisé de distribution de médicaments (100), le procédé comprenant :
la réception des données de transaction de distribution de médicaments associées au système automatisé de distribution de médicaments (104),
les données de transaction de distribution de médicaments comportant au moins un identifiant d'utilisateur, un identifiant de médicament, un identifiant de patient et une quantité distribuée d'un médicament associé à l'identifiant de médicament ;
la réception des données du dossier médical électronique correspondant aux données de transaction de distribution de médicaments, les données du dossier médical électronique comportant au moins un identifiant de patient, un médicament prescrit et une quantité administrée du médicament ;
la comparaison des données de transaction de distribution de médicaments aux données du dossier médical électronique correspondant ;
la détermination d'une quantité de médicament perdue prévue en fonction de la quantité de médicament distribuée et de la quantité de médicament administrée ;
la réception de données de dosage correspondant à un dosage quantitatif indiquant une quantité réelle de médicament perdue ;
la réception de données de transaction relatives à la perte de médicaments associées au système automatisé de distribution de médicaments, les données de transaction relatives à la perte de médicaments reçues indiquant d'une quantité de médicaments perdue ;
la comparaison de la quantité de médicament distribuée, la quantité de médicament perdue, la quantité de médicament administrée, la quantité attendue de médicament perdue et la quantité réelle de médicament perdue associées à un utilisateur avec les quantités de médicament distribuées, des quantités du médicament perdues, des quantités de médicament administrées, des quantités attendues du médicament perdues et des quantités réelles du médicament perdues associées à un ou plusieurs pairs de l'utilisateur dans un emploi similaire ;
l'identification de la variance indiquant un événement potentiel de détournement de médicaments sur la base de la comparaison ; et
la génération d'un ou de plusieurs rapports comportant au moins l'un des résultats de la comparaison et de l'écart identifié et comportant les résultats de l'analyse de la quantité de médicaments distribuée et/ou de la quantité de médicaments perdue au fil du temps sur en fonction d'au moins un parmi utilisateur, médicament, type d'utilisateur, lieu, délai, pratique, les résultats de l'analyse étant destinés à améliorer la sécurité des patients.

2. Procédé selon la revendication 1, dans lequel les données de transaction de distribution de médicaments comprennent des données de transaction relatives à la perte de médicaments ou des données de transaction de retour en stock de médicaments.

3. Procédé selon la revendication 1 comprenant en outre la réception de données de dosage correspondant à un dosage quantitatif pour un pourcentage prédéterminé de transactions de perte.

4. Procédé selon la revendication 1 comprenant en outre l'analyse des données de transaction de distribution de médicaments sur une période de temps afin d'identifier des tendances dans les données de transaction.

5. Procédé selon la revendication 1 comprenant en outre l'analyse d'au moins une partie des données de transaction de distribution de médicaments associées à un prestataire de soins de santé spécifique.

6. Procédé selon la revendication 1, comprenant en outre :
la réception des données d'administration de médicaments par perfusion indiquant la quantité réelle d'un médicament administré par un dispositif de perfusion ;
la réception des données de dosage correspondant à un dosage quantitatif indiquant une quantité réelle perdue de médicament pour perfusion ;
la détermination d'une quantité perdue attendue du médicament pour perfusion sur la base d'une quantité distribuée du médicament pour perfusion et de la quantité administrée du médicament ; et
l'identification de l'écart indiquant le détournement potentiel de médicaments en fonction de la quantité distribuée du médicament pour perfusion, de la quantité administrée du médicament pour perfusion, de la quantité perdue du médicament pour perfusion, de la quantité perdue attendue du médicament pour perfusion et de la quantité réelle perdue du médicament pour perfusion.

7. Système de vérification d'administration de médicaments (100), comprenant :
au moins un processeur (132) configuré pour :
recevoir des données de transaction d'administration de médicaments associées à une pluralité de transactions de distribution effectuées dans un système automatisé de distribution de médicaments (104), les données de transaction de distribution de médicaments comportant, pour chacune parmi la pluralité de transactions de distribution, un identifiant d'utilisateur, un identifiant de médicament, un identifiant de patient et une quantité de médicament distribuée ;
recevoir les données du dossier médical électronique correspondant aux données de transaction de distribution de médicaments, les données du dossier médical électronique comportant au moins un identifiant de patient, un médicament prescrit et une quantité administrée du médicament ;
recevoir des données de transaction relatives à la perte de médicaments associées à une pluralité de transactions de perte effectuées dans un système automatisé de distribution de médicaments, les données de transaction relatives à la perte de médicaments comportant, pour chacune parmi la pluralité de transactions de perte, un identifiant d'utilisateur, identifiant de médicament, identifiant de patient et une quantité de médicament perdue ;
pour chacune de la pluralité de transactions de distribution, comparer les données de transaction de distribution de médicaments aux données du dossier médical électronique correspondant ;
pour chacune de la pluralité de transactions de distribution, déterminer la quantité attendue de médicament perdue en fonction de la quantité de médicament distribuée et de la quantité de médicament administrée ;
pour chacune de la pluralité de transactions de perte, recevoir des données de dosage correspondant à un dosage quantitatif indiquant une quantité réelle de médicament perdue ;
comparer la quantité de médicament distribuée, la quantité de médicament perdue, la quantité de médicament administrée, la quantité attendue de médicament perdue et la quantité réelle de médicament perdue associées à un utilisateur avec des quantités de médicament distribuées, des quantités de médicament perdues, des quantités de médicament administrées, des quantités attendues de médicament perdues et des quantités réelles de médicament perdues associées à un ou plusieurs pairs de l'utilisateur dans un emploi similaire ;
identifier l'écart indiquant l'événement potentiel de détournement de médicaments sur la base de la comparaison, et
générer un ou plusieurs rapports comportant au moins l'un des résultats de la comparaison et de l'écart identifié et comportant les résultats de l'analyse de la quantité de médicaments distribuée et/ou de la quantité de médicaments perdue au fil du temps en fonction d'au moins l'un parmi utilisateur, médicament, type d'utilisateur, lieu, délai, pratique, les résultats de l'analyse étant destinés à améliorer la sécurité des patients.

8. Système de vérification d'administration de médicaments selon la revendication 7, dans lequel l'au moins un processeur est en outre configuré pour :
pour chacun de la pluralité des identifiants d'utilisateur, comparer un nombre d'écarts identifiés avec un nombre d'écarts identifiés associés à un groupe de référence correspondant à l'utilisateur.

9. Système de vérification d'administration de médicaments selon la revendication 7, dans lequel l'au moins un processeur est en outre configuré pour :
pour chacun de la pluralité d'identifiants d'utilisateur et pour chacune de la pluralité de transactions de distribution associées à l'identifiant d'utilisateur respectif, comparer la quantité de médicament distribuée à la quantité moyenne de médicament distribuée associée à un groupe de référence correspondant à l'utilisateur.

10. Système de vérification d'administration de médicaments selon la revendication 7, dans lequel l'au moins un processeur est en outre configuré pour :
pour chacune de la pluralité de transactions de perte, recevoir des données de dosage correspondant à un dosage quantitatif indiquant une quantité réelle de médicament perdue ; et
pour chacun de la pluralité d'identifiants d'utilisateur et pour chacune de la pluralité de transactions de perte, comparer la quantité réelle de médicament perdue à la quantité moyenne de médicament perdue associée à un groupe de référence correspondant à l'utilisateur

11. Système de vérification d'administration de médicaments selon la revendication 7, dans lequel l'au moins un processeur est en outre configuré pour :
recevoir des données relatives à l'administration de médicaments par perfusion associées à une pluralité de dispositifs de perfusion, les données relatives à l'administration de médicaments par perfusion comportant, pour chaque administration d'un médicament par perfusion par la pluralité de dispositifs de perfusion, un identifiant d'utilisateur, un identifiant de médicament par perfusion, identifiant de patient et une quantité réelle de médicament par perfusion administrée ;
recevoir des données de transaction de perte de médicaments pour perfusion associées à plusieurs transactions de perte de médicaments pour perfusion effectuées dans un système automatisé de distribution de médicaments, les données de transaction relatives à la perte de médicaments pour perfusion comportant, pour chacune de la pluralité de transactions de perte de médicaments pour perfusion, un identifiant d'utilisateur, un identifiant de médicament pour perfusion, un identifiant de patient et une quantité perdue d'un médicament pour perfusion ;
pour chaque administration d'un médicament par la pluralité de dispositifs de perfusion, comparer la quantité de médicament administrée à un troisième groupe de référence correspondant à l'identifiant d'utilisateur ;
pour chacune de la pluralité de transactions de perte, comparer la quantité de médicament perdue à un quatrième groupe de référence correspondant à l'identifiant d'utilisateur ; et
identifier un écart indiquant un détournement potentiel de médicament pour chacune des comparaisons qui satisfont à un seuil d'écart.
